# EUROPEAN PATENT SPECIFICATION

(11) **EP 0 539 492 B1**
(45) Date of publication and mention of the grant of the patent: **11.06.2003**
(21) Application number: 91913698.6
(22) Date of filing: 16.07.1991
(51) Int. Cl.: C07K 14/22, C12N 15/31, C12N 15/11, A61K 38/16, A61K 39/095, C12Q 1/00, C07K 16/12

(54) **ANTIGENIC IRON REPRESSIBLE PROTEINS FROM N. MENINGITIDIS RELATED TO THE HEMOLYSIN FAMILY OF TOXINS**
MIT DER FAMILIE DER HÄMOLYSIN-TOXINE VERWANDTE ANTIGENE EISEN-UBNTERDRÜCKENDE PROTEINE DES N. MENINGITIS
PROTEINES ANTIGENIQUES A ACTION LIMITEE PAR L'INCORPORATION DE FER TIREES DE LA BACTERIE N. MENINGITIDIS ET ASSOCIEES A LA FAMILLE DE TOXINES DES HEMOLYSINES

(30) Priority: 16.07.1990 US 552649
(43) Date of publication of application: 05.05.1993
(60) Divisional application: 03004818.5
(73) Proprietor: UNIVERSITY OF NORTH CAROLINA AT CHAPEL HILL, Chapel Hill, North Carolina 27514 (US)
(72) Inventor: SPARLING, P., Frederick, Moncure, NC 27559 (US); THOMPSON, Stuart, Alan, Carrboro, NC 27510 (US)
(74) Representative: Grünecker, Kinkeldey, Stockmair & Schwanhäusser Anwaltssozietät
(86) International application number: US9105014
(87) International publication number: WO92001460

(56) References cited:
- US-A- 4 681 761
- Journal of Experimental Medicine, Volume 165, issued April 1987, MIETZNER et al., "Purification and Characterization of the Major iron-regulated Protein expressed by Pathogenic Neisseriae", pages 1041-1057, see entire article.
- Infection and Immunity, Volume 51, No. 1, issued January 1986, MIETZNER et al., "Distribution of an Antigenically Related Iron-regulated Protein among the Neisseria spp", pages 60-68, see entire article.
- Infection and Immunity, Volume 55, No. 12, issued December 1987, STRATHDEE et al., "Extensive Homology between the Leukotoxin of Pasteurella haemolytica A1 and the Alpha-Hemolysin of Escherichia coli", pages 3233-3236, see entire document.
- Infection and Immunity, Volume 54, No. 3, issued December 1986, BLACK et al., "Human Immune Response to Iron-Repressible Outer Membrane Proteins of Neisseria Meningitidis", pages 710-713, see entire article.
- Infection and Immunity, Volume 58, No. 6, issued June 1990, SWIHART et al., "The HpmA Hemolysin is more Common than HLyA among Proteus Isolates", pages 1853-1860, see pages 1853, Discussion page 1858.
- Infection and Immunity, Volume 58, No. 9, issued September 1990, BANERJEE-BHATNAGAR et al., "Expression of Neisseria Meningitidis Iron-regulated Outer Membrane Proteins, Including a 70-Kilodalton Transferrin Receptor, and their potential for Use as Vaccines", pages 2875-2881, see entire document.
- Infection and Immunity, Volume 58, No. 9, issued September 1990, PETTERSSON et al., "Monoclonal antibodies against the 70-Kilodalton Iron-regulated Protein of Neisseria Meningitidis are Bactericidal and Strain specific", pages 3036-3041, see entire article.
- Infection and Immunity, Volume 57, No. 1, issued August 1989, MURAKAMI et al., "Cloning and Characterization of the Structural Gene for the Class 2 protein of Neisseria meningitidis", pages 2318-2323, see entire article.
- ABSTRACTS OF THE ANNUAL MEETING OF THE AMERICAN SOCIETY FOR MICROBIOLOGY 5 May 1991, WASHINGTON US page 31 A.L. LOBO ET AL. 'A survey of Gram-negative bacteria seeking new RTX family members and determination of relatedness among members using monoclonal antibodies'
- ABSTRACTS OF THE ANNUAL MEETING OF THE AMERICAN SOCIETY FOR MICROBIOLOGY 5 May 1991, WASHINGTON US page 32 S. THOMPSON ET AL. 'Cloning of a gene from Neisseria meningitidis with sequences similarity to the RTX family of cytotoxins

## Description

The present invention is directed to antigenic polypeptides isolated from Neisseria meninaitidis, antibodies raised against the polypeptides, vaccines containing the polypeptides and DNA encoding the polypeptides. The polypeptides are members of the hemolysin family of toxins, a typical member of which is alpha-hemolysin from E. coli.

Bacterial pathogenesis is a complicated and often poorly understood process. Many pathogenic bacteria secrete toxins that impair the metabolism and function of animal cells. Various classes of molecules constitute such toxins.

An example of a protein toxin is found in pathogenic E. coli strains that cause extra-intestinal infections in humans. Such infections are characterized by the lysis of mammalian erythrocytes. The hemolytic activity is due to a class of toxins known as hemolysin. The class includes alpha-hemolysin and beta-hemolysin; see Welch et al, Infection and Immunity 42, 178-186 (1983).

Another protein toxin is adenylate cyclase, which is found in Bordetella pertussis and Bacillus anthracis, and which impairs functions of professional phagocytes. These bacteria are responsible for whooping cough and anthrax, respectively.

A third class of protein toxins from pathogenic bacteria are the leukotoxins, which are found in Actinobacillus actinomycetemcomitans, which is the etiologic agent of localized juvenile periodontitis, and Pasteurella haemolvtica, which kills bovine leukocytes.

Interestingly, the adenylate cyclase from B. pertussis and B. anthracis and the leukotoxins from A. actinomycetemcomitans and P. haemolytica have amino acid sequences that exhibit considerable homology with that of alpha-hemolysin from E. Coli; see Glaser et al, Molecular Biology 2, 19-30 (1988) and Kolodrubetz et al, Infection and Immunity 57, 1465-1469 (1989). Apparently, there is a class of toxins found in various genera of bacteria. The amino acid sequence of this family of cytotoxins is characterized by a highly repeated nine amino acid motif, LxGGxGNDx, wherein x represents any amino acid. For the purposes of this specification, this family of toxins will be referred to as the hemolysin family of toxins.

It should be understood that "hemolysin family of toxins" is a generic name familiar to those in the art, and is not meant to imply that all members are hemolytic, or, for that matter, cytotoxic, although most are. Membership in the family depends on the existence of homology in the amino acid sequence, as defined below. In addition to those mentioned above, homologous proteins have also been found in Serratia and Proteus, although it is not certain whether these members of the hemolysin family are, in fact, cytotoxic.

Little is known about the intriguing and sometimes fatal bacteria Neisseria meningitidis, which is responsible for spinal meningitis and septic shock. N. meningitidis and the diseases it causes have been reviewed by Paterson in "Neisseria meningitidis and Meningococcal Disease" in Biologic and Clinical Basis of Infectious Diseases, W. B. Saunders Company, Chapter 43 (1980).

The genotype of N. meningitidis is very similar to that of N. gonorrhoeae, although the phenotype is quite different. It is often important to distinguish between these Neisseria species. Immunologic speciation is often difficult due to a lack of sufficient amounts of group-specific antigens.

N. meningitidis exists as various serotypes, the prevalence of which varies with time and location. The serotypes include A, B, C, D, X, Y, Z, 29-E and W-135.

The three most important known antigenic and/or toxic constituents of N. meningitidis infections are a capsular polysaccharide, a lipopolysaccharide-endotoxin cell wall complex and a Neisseria-specific protein. The capsular polysaccharide is a major _virulence factor that enables meningococci to resist phagocytosis by segmented neutrophils.

Vaccines containing meningococcal polysaccharides are used against some of the serotypes of N. meningitidis. For example, protection against the A, C, Y and W-135 serotypes is afforded by polysaccharide vaccines. Such vaccines are, however, inadequate for general protection against infection against N. meningitidis. For example, the immune response of serotypes A and C to polysaccharide vaccines is poor, especially in children under two years old, who constitute the group most susceptible to meningococcal disease. Moreover, no effective vaccine exists for serotype B, possibly because the group B capsular polysaccharide is relatively non-immunogenic.

It is apparent that much needs to be learned about the pathology of N. meningitidis. Possibly, additional understanding of this pathogen will lead to the discovery of useful vaccines in general for more serotypes than are currently available.

For example, it is not known why the colonization of the respiratory tract by N. meningitidis progresses to acute meningococcal disease and sometimes death in an occasional individual, whereas it does not do so in the great majority of others who are apparently at comparable risk. The amount of neither capsular polysaccharide nor lipopolysaccharide-endotoxin complex correlates with the seriousness of this disease. Exposure to microorganisms with antigenic constituents that cross-react with capsular polysaccharides of N. meningitidis has been proposed as an explanation; see Paterson, id.

Other explanations are also possible. For example, cross-immunity to antigens other than capsular polysaccharides cannot be ruled out. It is interesting to note in this regard that there are no known protein toxins associated with N. meningitidis. One reason for this may be that N. meningitidis is often cultured in vitro under iron-rich conditions that do not exist in a human host. It is known, however, that some meningococcal proteins are iron-repressed and are not observed in vitro, although they are expressed in vivo. See Black *et al*., Infection and Immunity 54, 710-713 (1986) and Brener et al., ibid. 33, 59-66 (1981). Further, a 70 Da iron-regulated outer-membrane protein (FeRP-70) from Neisseria Meningitidis is known from Ala'Aldeen *et al.* (J. Med. Microbiol., Vol. 32, May, 1990, p. 275-281).

One problem adressed by the present invention is the discovery of antigenic polypeptides and DNA sequences that are capable of identifying *N. meningitidis* and distinguishing it from *N. gonorroeae*. Another problem addressed by the present invention is the discovery of proteins capable of producing antibodies effective against meningococcal disease.

### SUMMARY OF THE INVENTION

These and other problems as will be apparent to those having ordinary skill in the art have been solved by providing an essentially pure, antigenic polypeptide having at least 900 amino acids comprising a segment of at least fifty amino acid residues and having an amino acid sequence present in *N. meningitidis*, wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

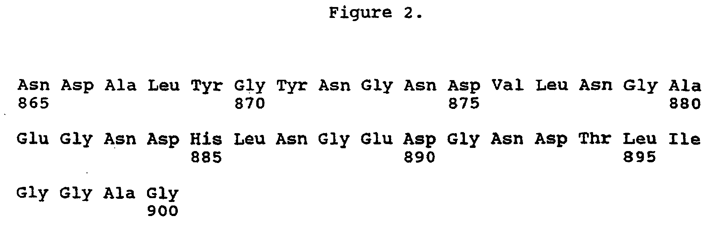

A preferred embodiment of the present invention is a polypeptide as explained above, wherein the segment has at least 100 amino acid residues, and more preferably, wherein the segment has at least 200 amino acid residues. Also a preferred embodiment of the present invention is a polypeptide as explained above, wherein the antigenic amino acid sequence is immunogenic. Further preferred is a polypeptide as explained above, wherein the amino acid sequence of the polypeptide comprises the sequence shown in Figure 2. Additionally, one embodiment of the present invention provides a polypeptide as explained above, wherein antibodies against the polypeptide cross-react with at least one other member of the hemolysin family of toxins from other genera of bacteria. The other genera of bacteria preferably include *Escherichia*, *Serratia, Pasteurella, Proteus, Actinobacillus*, and *Bordetella.* More preferably, the other members of the hemolysin family of toxins comprise alpha-hemolysin from *Escherichia coil;* leukotoxin from *Actinobacillus actinomycetemcomitans;* leukotoxin from *Pasteurella haemolytica;* adenylate cyclase from *Bordetella pertussis* and adenylate cyclase from *Bacillus anthraces*. Most preferably the other member of the hemolysin family of toxins is alpha-hemolysin from *E. coli.*

A further embodiment of the present invention provides a method of producing an antigen useful in protecting a mammal from infection by *N. meningitidis* comprising the steps of (a) preparing an essentially pure, antigenic polypeptide having at least 900 amino acids comprising a segment having at least fifty amino acid residues, wherein the amino acid sequence is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue, and (b) rendering the polypeptide non-toxic to mammals. Preferably, one embodiment provides a method of producing an antigen preferably as explained above, wherein the amino acid sequence is present in a polypeptide found in *N. meningitidis.* More preferably, one embodiment provides a method of producing an antigen preferably as explained above, wherein the mammal is a human.

An other embodiment of the invention is a method of producing a vaccine composition useful in protecting a mammal from infection by *N. meningitidis* comprising the steps of: (a) preparing an essentially pure, antigenic polypeptide having at least 900 amino acids comprising a segment having at least fifty amino acid residues, wherein the amino acid sequence is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue, (b) rendering the polypeptide non-toxic to mammals, and (c) combining the non-toxic polypeptide with a pharmaceutically acceptable carrier. In a preferred embodiment the polypeptide is isolated from *N. menigitidis*. In another preferred embodiment the mammal is a human.

One embodiment of the invention provides a vaccine composition capable of immunizing mammals against infections by *N. meningitidis,* the vaccine composition comprising: (a) an immunogenic polypeptide having at least 900 amino acids that is non-toxic to mammals and comprises a segment having at least fifty amino acid residues, wherein the amino acid sequence is different from, but substantially homologous with, the hemolysin family of toxins, or antigenic fragments of such polypeptides, wherein the antigenic fragments comprise at least three repeats of the hemolysin consensus sequence LXGGXGNDX, wherein X, independently, represents any single amino acid residue, and (b) a pharmaceutically acceptable carrier. Preferably the invention provides a vaccine composition of as explained above, wherein the polypeptide is present in *N. meningitidis*. More preferably the polypeptide is present in outer membranes of *N. meningitidis*. Another preferred embodiment is a vaccine composition as explained above, wherein the mammal is a human.

Further, one embodiment of the present invention are monoclonal antibodies raised against a polypeptide having at least 900 amino acids comprising a segment of at least fifty amino acid residues and having an amino acid sequence present in *N. meningitidis,* wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

Another embodiment of the present invention is an isolated nucleic acid molecule that encodes a polypeptide having at least 900 amino acids comprising a segment of at least fifty amino acid residues and having an amino acid sequence present in *N. meningitidis,* wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

One embodiment of the present invention is a method of detecting the presence of antibodies specific for a member of the hemolysin family of toxins in a sample comprising the steps of: (a) preparing an essentially pure, antigenic polypeptide having at least 900 amino acids comprising a segment having at least fifty amino acid residues and wherein the polypeptide comprises an amino acid sequence present in *N. meningitidis*, wherein the amino acid sequence of the segment is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue, and (b) determining whether the polypeptide recognizes an antibody in the sample.

Another further embodiment of the present. invention is an essentially pure polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of: L at position 1, G at position 3, G at position 4, G at position 6, N at position 7, D at position 8, and x at positions 2, 5 and 9, wherein x represents any single amino acid residue, and antigenic fragments of such polypeptides, wherein the antigenic fragments comprise at least three repeats of the hemolysin consensus sequence LXGGXGNDX, wherein X, independently, represents any single amino acid residue. A preferred embodiment is a polypeptide as explained above, wherein the segment has at least 100 amino acid residues, and more preferably, wherein the segment has at least 200 amino acid residues. Also a preferred embodiment is the polypeptide as explained above, wherein the antigenic amino acid sequence is immunogenic. More preferred is the polypeptide as explained above, wherein antibodies against the polypeptide cross-react with at least one other member of the hemolysin family of toxins from other genera of bacteria. Preferably the other genera of bacteria include *Escherichia*, *Serratia*, *Pasteurella*, *Proteus*, *Actinobacillus*, and *Bordetella*. More preferably the other members of the hemolysin family of toxins comprise alpha-hemolysin from *Escherichia coli*, leukotoxin from *Actinobacillus actinomycetemcomitans*, leukotoxin from *Pasteurella haemolytica*, adenylate cyclase from *Bordetella pertussis* and adenylate cyclase from *Bacillus anthracis*. Most preferably the other member of the hemolysin family of toxins is alpha-hemolysin from *E. coli.*

Another embodiment of the present invention is a method of producing an antigen useful in protecting a mammal from infection by *N. meningitidis* comprising the steps of: (a) preparing an essentially pure polypeptide having at least 900 amino acids comprising a segment having an amino acid sequence that consists of at least three repeats of the nine amino acid hemolysin consensus sequence, the hemolysin consensus sequence consisting of at least four of: L at position 1, G at position 3 G at position 4, G at position 6, N at position 7, D at position 8, and x at positions 2, 5 and 9, wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue, and (b) rendering the polypeptide or fragment non-toxic to mammals. A preferred embodiment is a method as explained above, wherein the amino acid sequence is present in a polypeptide found in *N. meningitidis*.

One further embodiment of the present invention also provides a method of producing a vaccine composition useful in protecting a mammal from infection by *N. meningitidis* comprising the steps of: (a) preparing an essentially pure, antigenic polypeptide having at least 900 amino acids comprising a segment having an amino acid sequence that consists of at least three repeats of the nine amino acid hemolysin consensus sequence, the hemolysin consensus sequence consisting of at least four of: L at position 1, G at position 3, G at position 4, G at position 6, N at position 7; D at position 8, and x at positions 2, 5 and 9, wherein x represents any single amino acid residue, or antigenic fragments of such polypeptides, wherein the antigenic fragments comprise at least three repeats of the hemolysin consensus sequence LXGGXGNDX, wherein X independently, represents any single amino acid residue, and (b) rendering the polypeptide non-toxic to mammals, and (c) combining the non-toxic polypeptide with a pharmaceutically acceptable carrier. In a preferred embodiment, the the polypeptide is isolated from *N. meningitidis*.

A vaccine composition capable of immunizing mammals against infections by *N. meningitidis*, the vaccine composition comprising: (a) an essentially pure, immunogenic polypeptide having at least 900 amino acids comprising a segment having an amino acid sequence that consists of at least three repeats of the nine amino acid hemolysin consensus sequence, the hemolysin consensus sequence consisting of at least four of: L at position 1, G at position 3, G at position 4, G at position 6, N at position 7, D at position 8, and x at positions 2, 5 and 9, wherein x represents any single amino acid residue, or antigenic fragments of such polypeptides, wherein the antigenic fragments comprise at least three of the hemolysin consensus sequence LXGGXGNDX, wherein X, independently, represents any single amino acid residue, and (b) a pharmaceutically acceptable carrier. In a preferred embodiment, the polypeptide is present in *N. meningitidis*. More preferably, the polypeptide is present in outer membranes of *N. meningitidis*. In an other preferred embodiment the mammal is a human.

A further embodiment of the present invention are monoclonal antibodies raised against an essentially pure polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of: L at position 1, G at position 3, G at position 4, G at position 6, N at position 7, D at position 8, and x at positions 2, 5 and 9, wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue.

An other embodiment of the present invention is an isolated nucleic acid molecule that encodes a polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of: L at position 1, G at position 3, G at position 4, G at position 6, N at position 7, D at position 8, and x at positions 2, 5 and 9, wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue.

Also, one embodiment of the present invention is a method of detecting the presence of *N. meningitidis* in a sample comprising the steps of: (a) preparing a probe that recognizes a polypeptide that is a member of the hemolysin family of toxins or a fragment thereof, or a nucleic acid molecule encoding the polypeptide or fragment wherein the polypeptide that is a member of the hemolysin family of toxins or nucleic acid molecule encoding the polypeptide is present in *N. meningitidis;* and (b) determining whether the probe recognizes *N. meningitidis* in the sample. In a preferred embodiment the probe is an antibody. In a more preferred embodiment the antibody is monoclonal. In another preferred embodiment the probe is a nucleic acid molecule. As a further, one embodiment of the present invention is a method of detecting the presence of antibodies specific for a member of the hemolysin family of toxins in a sample comprising the steps of: (a) preparing an antigenic polypeptide having at least 900 amino acids and wherein the polypeptide comprises an amino acid sequence present in *N. meningitidis* comprising a segment having an amino acid sequence that consists of at least three repeats of the nine amino acid hemolysin consensus sequence, the hemolysin consensus sequence consisting of at least four of: L at position 1, G at position 3, G at position 4, G at position 6, N at position 7, D at position 8, and x at positions 2, 5 and 9, wherein x represents any single amino acid residue; and (b) determining whether the polypeptide recognizes an antibody in the sample, or antigenic fragments of such polypeptides, wherein the antigenic fragments comprise at least three repeats of the hemolysin consensus sequence LXGGXGNDX, wherein X, independently, represents any single amino acid residue.

The invention further includes antigenic fragments of such polypeptides, antibodies raised against such polypeptides, nucleotide sequences encoding such polypeptides, and vaccines containing such polypeptides.

### DETAILED DESCRIPTION OF THE INVENTION

### The Polypeptide Segment

It has unexpectedly been discovered that when N. meningitidis is grown under iron-limiting conditions, a polypeptide comprising a segment having an amino acid sequence that is different from, but substantially homologous with, a segment of the hemolysin family of toxins is expressed. Monoclonal antibodies raised against the polypeptide found in N. meningitidis, such as A4.85 (see below), cross-react in Western blots with alpha-hemolysin (HlyA) produced by the hlyA gene in E. coli and adenylate cyclase produced in Bordetella pertusssis.

The hemolysin family of toxins, as used herein, includes the homologous, cytotoxic or proteolytic polypeptides found in bacteria of the genera Escherichia, Serratia, Pasteurella, Proteus, Actinobacillus, and Bordetella. The family specifically includes alpha-hemolysin, leukotoxin, and adenylate cyclase.

Determinations whether two amino acid sequences are substantially homologous are, for the purpose of the present specification, based on PASTA searches in accordance with Pearson and Lipman, Proc. Natl. Acad. Sci. USA 85, 2444-2448 (1988). A substantially homologous sequence in accordance with the present invention has at least 15% identity, preferably at least 20% identity and, more preferably, at least 25% identity in amino acid sequence when determined in accordance with the method of Pearson and Lipman.

The polypeptide of the present invention need not contain a segment that is identical to other members of the hemolysin family of toxins. The identity in accordance with the FASTA method may be as high as 90% or 95%, but when isolated from N. meningitidis normally does not exhibit identities greater than 40% or 50%.

The size of the polypeptide is not critical, as long as it has at least 900 amino acids and contains a segment that is substantially homologous to a segment of a member of the hemolysin family of toxins. The substantially homologous segment has at least fifty amino acid residues, preferably at least 100 amino acid residues, and more preferably at least 200 amino acid residues. In this specification, the word "polypeptide" will be considered indistinguishable from words like "protein" and "peptide."

The segment of the meningococcal polypeptide that is substantially homologous to a segment of the hemolysin family of toxins contains the same nine amino acid motif that is characteristic of all the members of the hemolysin family of toxins. The consensus sequence is LxGGxGNDx, hereinafter hemolysin consensus sequence. The amino acid represented by x may be any single amino acid.

The polypeptide of the invention may be defined in terms of the hemolysin consensus sequence as well as by the substantial homology standard described above. For this purpose, a nine amino acid sequence is considered to be a hemolysin consensus sequence if it contains at least four, preferably at least five, and more preferably all six of the specifically defined amino acid residues (i.e. L-GG-GND-) at the correct position.

Referring to Figure 2, which, as described below, is a partial polypeptide isolated from N. meningitidis, the homologous segment comprises three stretches of multiple repeats of the hemolysin consensus sequence, i.e. between amino acids 486 and 512, between amino acids 623 and 712, and from 823 to the end. There are 21 complete consensus sequences in Figure 2.

The polypeptides of the present invention contain segments that are present in N. meningitidis and that comprise at least three, preferably at least five, and more preferably at least ten hemolysin consensus sequences. The polypeptides of the invention may have as many as at least 21 hemolysin consensus sequences.

The polypeptide is isolated, which means that it is essentially free of other proteins, especially of other proteins from N. meningitidis. Essentially free from other proteins means that it is at least 90%, preferably at least 95% and, more preferably, at least 98% free of other proteins.

Preferably, the polypeptide is essentially pure, which means that the polypeptide is free not only of other polypeptides, but also of other materials used in the isolation and identification of the polypeptide, such as, for example, sodium dodecyl sulfate and other detergents as well as nitrocellulose paper. The polypeptide is at least 90% free, preferably at least 95% free and, more preferably, at least 98% free of such materials.

The polypeptide of the present invention is antigenic, which means that the polypeptide induces specific antibodies in a mammal. Preferably, the polypeptide is immunogenic.

The polypeptide may be the entire polypeptide as it exists in N. meningitidis, or an antigenic, preferably immunogenic, fragment of the whole polypeptide. Antigenic and/or immunogenic fragments of antigenic and/or immunogenic polypeptides may be identified by methods known in the art. Usually, the antigenic fragment will comprise at least a portion of the segment having an amino acid sequence that is different from, but homologous, to the amino acid sequence of a segment of a polypeptide that is a member of the hemolysin family of toxins, or will comprise at least a portion of the segment having at least three, preferably at least five, and more preferably at least ten hemolysin consensus sequences.

### Preparation of the Polypeptide

The polypeptides of the present invention may be prepared by methods known in the art. Such methods include isolating the polypeptide directly from N. meningitidis; isolating or synthesizing DNA encoding the polypeptide and using the DNA to produce recombinant polypeptide; and synthesizing the polypeptide from individual amino acids.

The polypeptide or DNA encoding the polypeptide may be isolated from any serotype of N. meningitidis. Such serotypes include A, B, C, D, X, Y, Z, 29-E and W-135.

Suitable sources of meningococcal strains from which the polypeptide and DNA encoding the polypeptide may be isolated are available. Such sources include the American Type Culture Collection (Bethesda, MD) and the Neisseria Repository (NAMRU, University of California, Berkeley). Suitable strains include FAM18 and FAM20 (Dyer et al, Microbial Pathogenesis 3, 351-363 (1987)), and FAM 19. Additional meningococcal strains are described by Schryvers and Morris in Infection and Immunity 56, 1144-1149 (1988).

The polypeptide may be isolated directly from N. meningitidis by methods known in the art. First, meningococcal outer membranes are isolated and prepared by known methods. The methods described by West and Sparling in Infect. Immun. 47, 388-394 (1985) and by Schryvers and Morris in Infect. Immun. 56, 1144-1149 (1988) are suitable.

The isolated membrane proteins may be solubilized by known methods, such as the addition of detergents. Commonly used detergents include Octyl-B-Glucoside, Chaps, Zwittergent 3.14 or Triton-X. The use of detergents to enhance solubility of membrane proteins is described by Jones et al. in Finby, Solubilization and Reconstitution of Membrane Proteins: A Practical Approach, IRL Press (1986), Helenius et al. in Biochim. Biophys. Acta 415, 29 (1975) and Hjelmeland and Chrambach, Methods Enzymol. 104, 305 (1984).

Proteins are isolated from the solubilized membrane fraction by standard methods. Some suitable methods include precipitation and liquid chromatographic protocols such as ion exchange, hydrophobic interaction and gel filtration. See, for example, Methods Enzymol. 182 (Guide to Protein Chemistry, Deutscher, Ed. Section VII) 309 (1990) and Scopes, Protein Purification. Springer-Verlag, New York (1987).

Alternatively, purified material is obtained by separating the protein on preparative SDS-PAGE gels, slicing out the band of interest and electroeluting the protein from the polyacrylamide matrix by methods known in the art. The detergent SDS is removed from the protein by known methods, such as by dialysis or the use of a suitable column, such as the Extracti-Gel® column from Pierce.

The polypeptide may also be produced by isolating DNA that encodes the polypeptide; cloning the DNA in a suitable host; expressing the DNA in the host; and harvesting the polypeptide.

The first DNA encoding the polypeptide of the present invention was isolated by an immunoscreening method. Such methods are described by Maniatis et al in "Molecular Cloning: A Laboratory Manual," Cold Spring Harbor Laboratory Press, Cold spring Harbor, New York (1982).

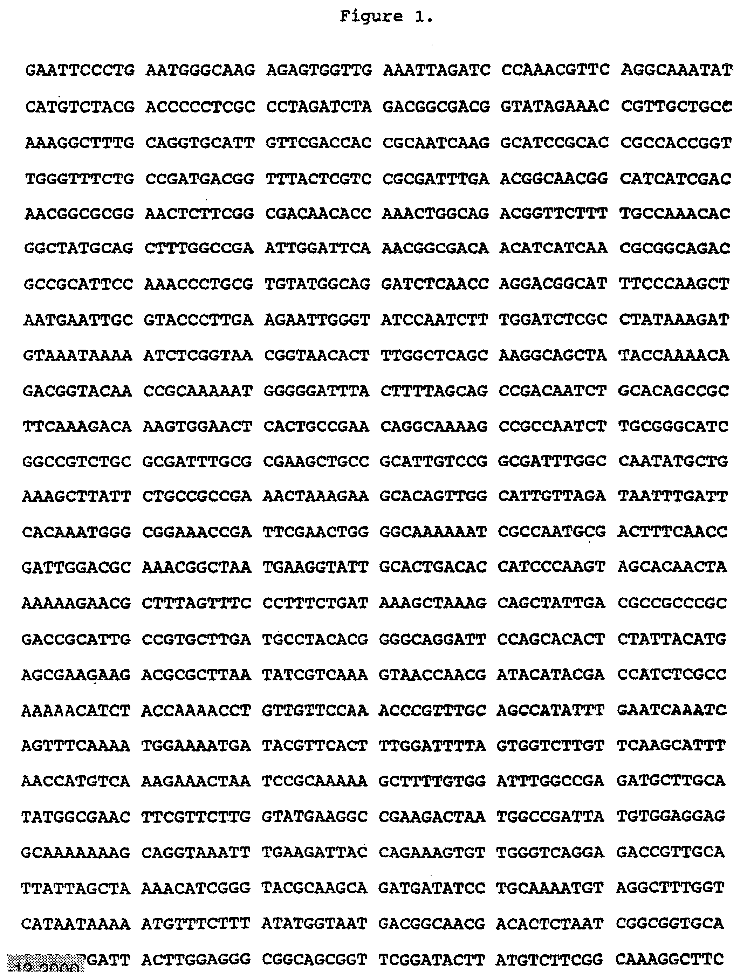

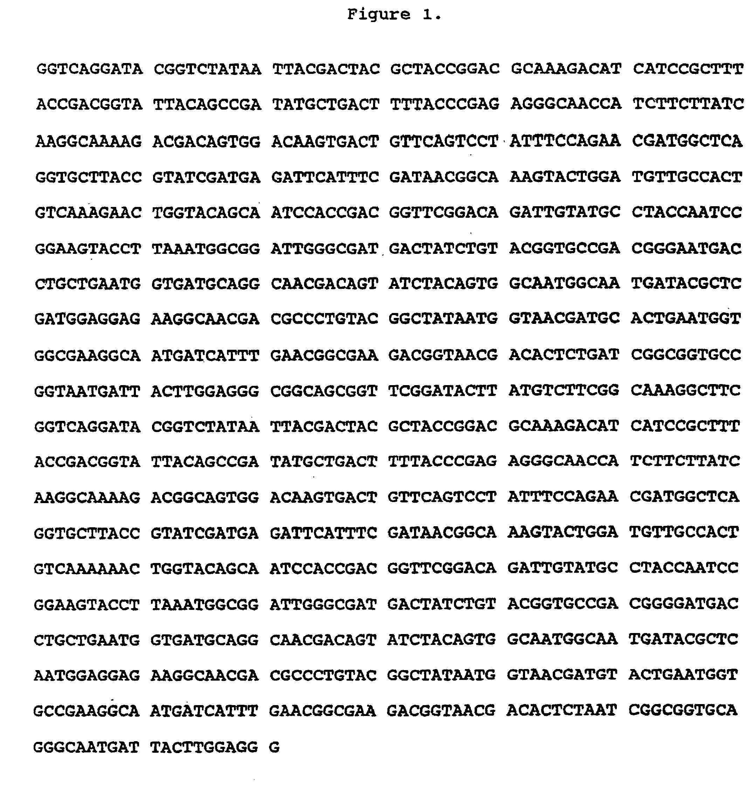

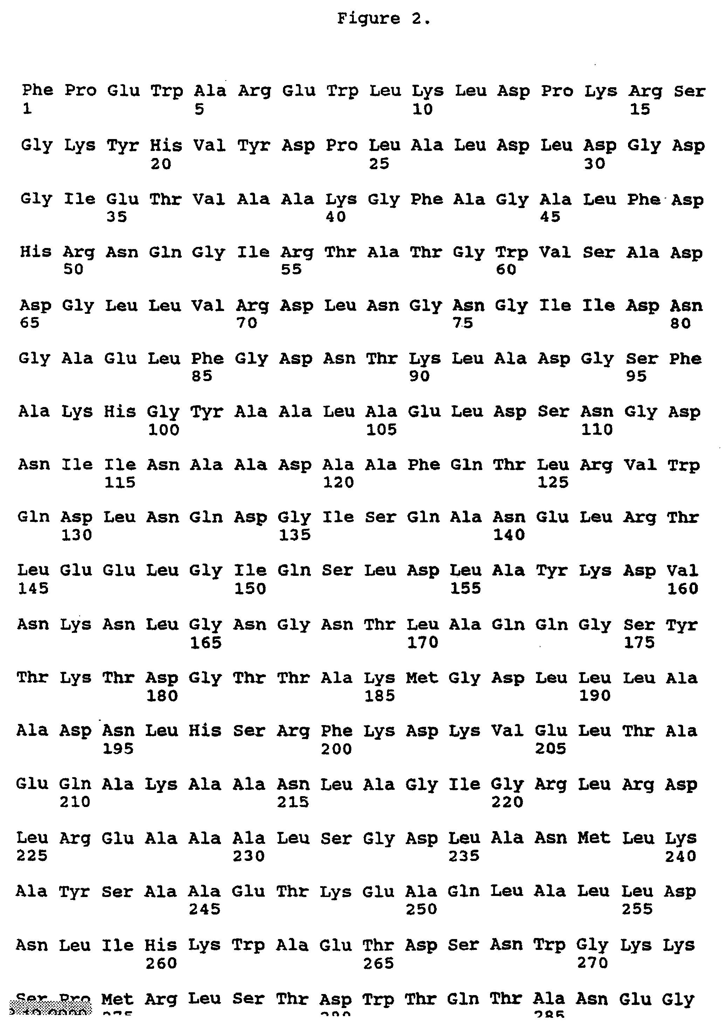

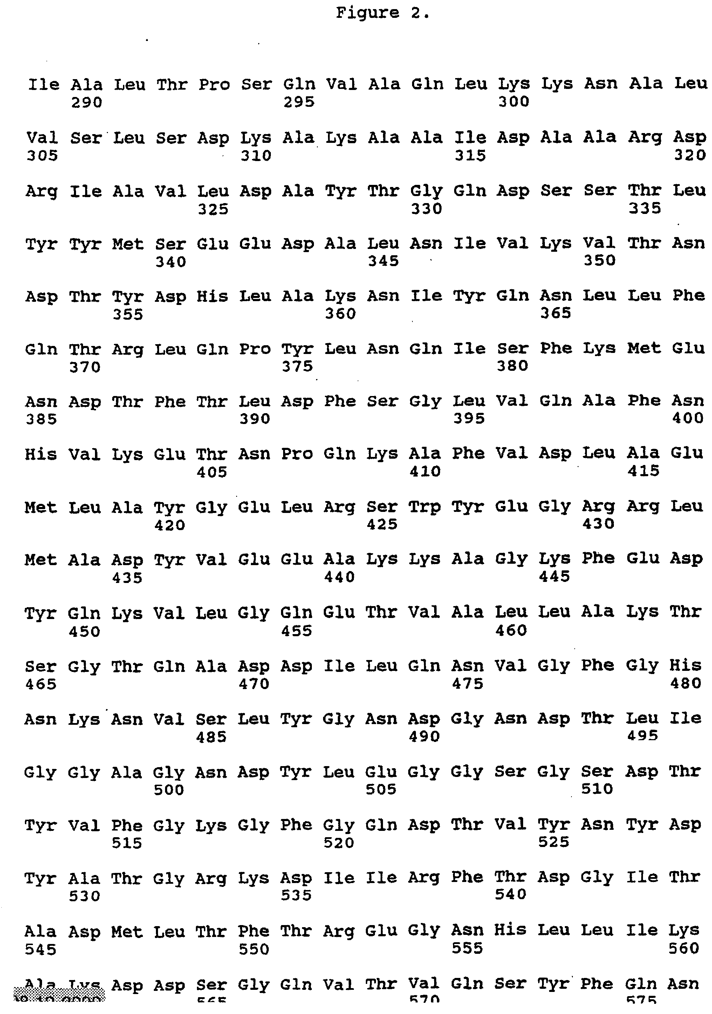

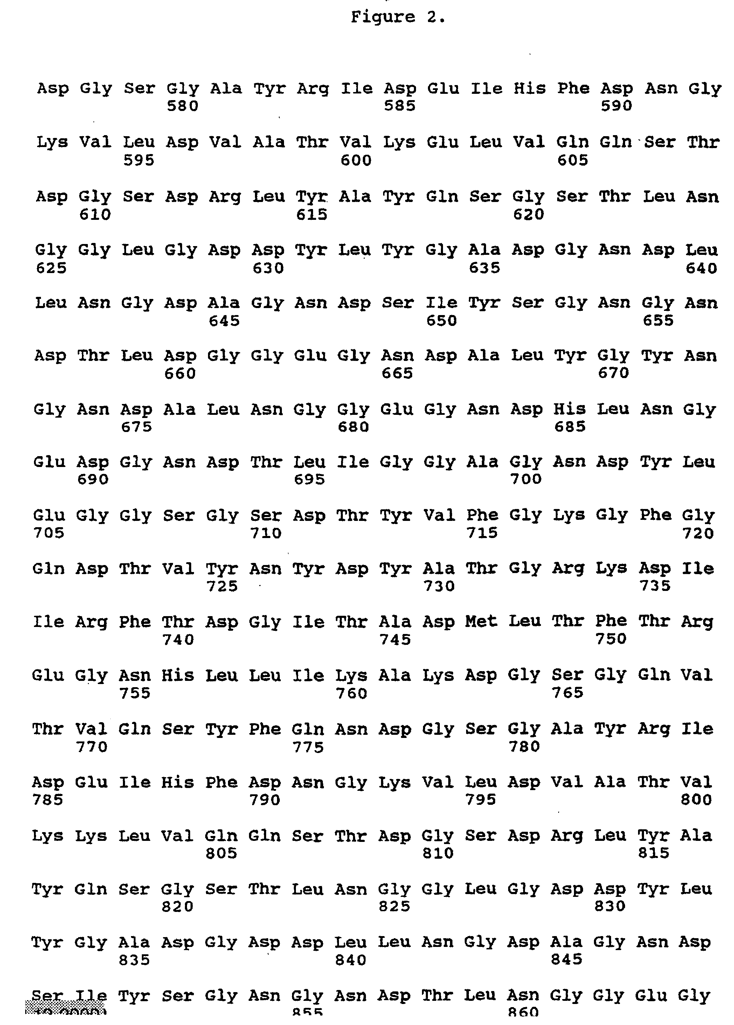

Briefly, monoclonal antibodies were generated against iron-stressed outer membrane proteins of N. meningitidis strain FAM20. One monoclonal antibody, A4.85, recognized several iron-regulated proteins in Western blots of the FAM20 outer membranes. A4.85 was used to isolate a clone from an FAM20 genomic library constructed in the expression vector lambda-gt11. The sequence of this clone was determined and used to clone adjacent genomic restriction fragments. The adjoined DNA sequence of this region contained a long open reading frame, which is shown as Figure 1. The amino acid sequence predicted from the nucleotide sequence of Figure 1 is shown as Figure 2.

A FASTA homology search in accordance with Pearson and Lipman, Proc. Natl. Acad. Sci. USA 85, 2444-2448 (1988) of the amino acid sequence deduced from the open reading frame (Figure 2) was performed. Surprisingly, the amino acid sequence exhibited substantial homology to several members of the hemolysin family of toxins, as ' discussed above.

As further evidence that the polypeptide isolated from N. meningitidis is a member of the hemolysin family of toxins, the antibody raised against, and used to isolate, the polypeptide shown as Figure 2, A4.85, cross-reacted strongly with alpha-hemolysin (HlyA) from E. coli and with adenylate cyclase produced in Bordetella pertusssis.

The immunoscreening method may be repeated in order to obtain additional fragments of the gene encoding the polypeptide of the invention or to obtain the gene encoding the entire polypeptide. It is, of course, not necessary to repeat the immunoscreening process. The entire gene or additional fragments of the gene are preferably isolated by using the known DNA sequence or fragments thereof as a probe. To do so, meningococcal DNA restriction fragments, either flanking the ends of the region already cloned or containing the entire region, are identified by Southern hybridization using labelled oligonucleotide probes derived from a previously determined sequence, such as that shown as Figure 1, or a fragment thereof.

The DNA obtained may be amplified by methods known in the art. One suitable method is the polymerase chain reaction (PCR) method described by Mullis et al in U.S. Patent 4,683,195 and by Sambrook, Fritsch and Maniatis (eds) in Molecular Cloning, A Laboratory Manual, Second Edition, cold Spring Harbor Laboratory Press (1989). It is convenient to amplify the clones in the lambda-gt11 vectors using lambda-gt11-specific oligomers as the amplimers (available from Stratagene).

The restriction fragments are cloned into a suitable vector, such as a plasmid or bacteriophage, and sequenced in accordance with methods known in the art. A suitable sequencing method is the dideoxy chain terminating method described by Sanger et al in Proc. Natl. Acad. Sci. USA 74, 5463-5467 (1977). Suitable vectors and polymerases for sequencing are known. A suitable vector is the Bluescript® vector of Stratagene. A suitable polymerase is Sequenase® (United States Biochemical Corp., Cleveland, OH).

The DNA encoding the polypeptide of the invention may be used to express recombinant polypeptide in a wide variety of host cells using a wide variety of vectors. The host may be prokaryotic or eukaryotic. The DNA may be obtained from natural sources and, optionally, modified. The genes may also be synthesized in whole or in part.

Cloning vectors may comprise segments of chromosomal, non-chromosomal and synthetic DNA sequences. Some suitable prokaryotic vectors include plasmids from E. coli, such as colE1, pCR1, pBR322, pMB9, and RP4. Prokaryotic vectors also include derivatives of phage DNA such as M13, fd, and other filamentous single-stranded DNA phages.

Vectors for expressing proteins in bacteria, especially E.coli, are also known. Such vectors include pK233 (or any of the tac family of plasmids), T7, and lambda P_{L}. Examples of vectors that express fusion proteins include the PATH vectors described by Dieckmann and Tzagoloff in J. Biol. Chem. 260, 1513-1520 (1985). These vectors contain DNA sequences that encode anthranilate synthetase (TrpE) followed by a polylinker at the carboxy terminus. Other expression vector systems are based on beta-galactosidase (pEX); maltose binding protein (pMAL); and glutathione S-transferase (pGST) - see Gene 67, 31 (1988) and Peptide Research 3, 167 (1990).

Vectors useful in yeast are available. A suitable example is the 2u plasmid.

Suitable vectors for use in mammalian cells are also known. Such vectors include well-known derivatives of SV-40, adenovirus, retrovirus-derived DNA sequences and vectors derived from combination of plasmids and phage DNA.

Further eukaryotic expression vectors are known in the art (e.g., P.J. Southern and P. Berg, J. Mol. Appl. Genet. 1, 327-341 (1982); S. Subramani et al, Mol. Cell. Biol. 1, 854-864 (1981); R.J. Kaufmann and P.A. Sharp, "Amplification And Expression Of Sequences Cotransfected with A Modular Dihydrofolate Reductase Complementary DNA Gene," J. Mol. Biol. 159, 601-621 (1982); R.J. Kaufmann and P.A. Sharp, Mol. Cell. Biol. 159, 601-664 (1982); S.I. Scahill et al, "Expression And Characterization Of The Product Of A Human Immune Interferon DNA Gene In Chinese Hamster Ovary Cells," Proc. Natl. Acad. Sci. USA 80, 4654-4659 (1983); G. Urlaub and L.A. Chasin, Proc. Natl. Acad. Sci. USA 77, 4216-4220, (1980).

Useful expression hosts include well-known prokaryotic and eukaryotic cells. Some suitable prokaryotic hosts include, for example, E. coli, such as E. coli SG-936, E. coli HB 101, E. coli W3110, E. coli X1776, E. coli X2282, E. coli DHI, and E. coli MRCl, Pseudomonas, Bacillus, such as Bacillus subtilis, and Streptomyces. Suitable eukaryotic cells include yeasts and other fungi, insect, animal cells, such as COS cells and CHO cells, human cells and plant cells in tissue culture.

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the lac system, the trp system, the tac system, the trc system, major operator and promoter regions of phage lambda, the control region of fd coat protein, the glycolytic promoters of yeast, e.g., the promoter for 3-phosphoglycerate kinase, the promoters of yeast acid phosphatase, e.g., Pho5, the promoters of the yeast alpha-mating factors, and promoters derived from polyoma, adenovirus, retrovirus, and simian virus, e.g., the early and late promoters or SV40, and other sequences known to control the expression of genes of prokaryotic or eukaryotic cells and their viruses or combinations thereof.

The recombinant polypeptide is purified by methods known in the art. Suitable methods are described F. A. O. Marston, "The Purification of Eukaryotic Polypeptides Expressed in Escherichia coli," in DNA Cloning, D. M. Glover, Ed., Vol. III, IRL Press Limited, England (1987).

The polypeptide of the invention and DNA encoding the polypeptide may also be chemically synthesized from individual amino acid residues and nucleotides, respectively, by methods known in the art. Suitable methods for synthesizing the polypeptide are described by Stuart and Young in "Solid Phase Peptide Synthesis," Second Edition, Pierce Chemical Company (1984). Suitable methods for synthesizing DNA are described by Caruthers in Science 230, 281-285 (1985).

### VACCINES

A polypeptide comprising a segment having an amino acid sequence that is different from, but substantially homologous with, the amino acid sequence of a member of the hemolysin family of toxins is, unexpectedly, an antigen useful for protecting a mammal from infectious diseases caused by N. meningitidis. The mammal is typically a human.

To be useful, the antigen is non-toxic to the mammal being immunized. If the antigen is toxic, it may be detoxified by methods known in the art. Such methods include, for example, providing antigenic, non-toxic fragments of the entire polypeptide or detoxifying a polypeptide by, for example, binding the toxin to a carrier molecule that destroys toxicity, but does not affect antigenicity. The carrier molecule is typically another polypeptide.

Preferably, an amino acid sequence of the antigen is present in a polypeptide found in N. meningitidis. The polypeptide or non-toxic, antigenic fragments useful in immunizing mammals may be made by methods known in the art, such as by isolation from N. meningitidis, production by recombinant DNA techniques, or chemical synthesis, as described above.

The length of the fragment is not critical as long as the fragment has at least 900 amino acids and is antigenic and non-toxic. Therefore, the fragment should contain sufficient amino acid residues to define the epitope. Methods for isolating and identifying antigenic fragments from known antigenic polypeptides are described by Salfeld et al. in J. Virol. 63, 798-808 (1989) and by Isola et al. in J. Virol. 63, 2325-2334 (1989).

If the fragment defines the epitope, but is too short to be antigenic, it may be conjugated to a carrier molecule. Some suitable carrier molecules include keyhole limpet hemocyanin and bovine serum albumen. Conjugation may be carried out by methods known in the art. One such method is to combine a cysteine residue of the fragment with a cysteine residue on the carrier molecule.

The present invention further includes vaccine compositions for immunizing mammals, including humans, against infection by N. meningitidis. The vaccine comprises an immunogenic antigen as described above in a suitable carrier. Suitable carriers include any of the standard pharmaceutically acceptable carriers, such as water, phosphate buffered saline solution, and emulsions.

The vaccine may include adjuvants, such as muramyl peptides, and lymphokines, such as interferon, interleukin-1 and interleukin-6. The antigen may be adsorbed on suitable particles, such as aluminum oxide particles, or encapsulated, in liposomes, as is known in the art.

Vaccines may be administered to a mammal by methods known in the art. Such methods include, for example, intravenous, intraperitoneal, subcutaneous, or intramuscular administration.

### Antibodies

The present invention provides antibodies raised against a polypeptide of the invention. The polypeptide comprises an amino acid sequence that defines an epitope, and is different from, but substantially homologous with the amino acid sequence of a segment of a member of the hemolysin family of toxins. The antibodies are preferably raised against a polypeptide comprising an amino acid sequence that is present in N. meningitidis, and that is different from polypeptides that are members of the hemolysin family of toxins from other genera of bacteria.

The antibodies are preferably monoclonal. Monoclonal antibodies may be produced by methods known in the art. These methods include the immunological method described by Kohler and Milstein in Nature 256, 495-497 (1975) and the recombinant DNA method described by Huse et al in Science 246, 1275-1281 (1989).

Mammals, including humans, suffering from diseases caused by infection with N. meningitidis may be treated by administering antibodies specific to a member of the hemolysin family of toxins. Antibodies raised against a member of the hemolysin family of toxins from any genera of bacteria are suitable, although antibodies raised against a polypeptide comprising an amino acid sequence present in N. meningitidis is preferred.

For therapeutic purposes, it is necessary for the antigenic polypeptides of the invention to produce neutralizing antibodies. Neutralizing antibodies are antibodies that significantly inhibit the growth of or kill the bacterial cells and/or significantly neutralize the toxin function of the polypeptide in vitro or in vivo. Growth of the bacteria is significantly inhibited or the toxin function of the polypeptide is significantly neutralized in vivo if the inhibition or neutralization is sufficient to prevent or reduce the symptoms of the disease of a mammal infected with the disease.

Neutralizing antibodies may also be used to produce anti-idiotypic antibodies useful as vaccines for immunizing mammals, including humans, suffering from diseases caused by infection with N. meningitidis. Anti-idiotypic antibodies are prepared in accordance with methods known in the art.

### NUCLEIC ACID MOLECULES

The present invention also includes isolated nucleic acid molecules that encode any of the polypeptides of the invention described above. The nucleic acid molecule may be DNA or RNA.

The utility of the nucleic acid molecule lies in its ability to be used as a probe for detecting N. meningitidis, as explained below, or to produce a polypeptide of the invention, as explained above. The nucleic acid molecule may be prepared by methods known in the art. Suitable methods include isolating the DNA from N. meningitidis or synthesizing the DNA in accordance with known procedures as described above.

### PROBES

The present invention further provides a method of detecting the presence of N. meningitidis in a sample. The method involves use of a probe that recognizes a polypeptide that is a member of the hemolysin family of toxins, and, in particular, a member of the hemolysin family of toxins present in N. meningitidis, or a gene encoding such a polypeptide. The probe recognizes N. meningitidis if present in the sample.

The probe may be an antibody, preferably a monoclonal antibody. The antibodies may be prepared as described above.

Methods are known for detecting polypeptides with antibodies. For example, a polypeptide may be immobilized on a solid support. Immobilization of the polypeptide may occur through an immobilized first antibody specific for the polypeptide. The immobilized first antibody is incubated with a sample suspected of containing the polypeptide. If present, the polypeptide binds to the first antibody.

A second antibody, also specific for the polypeptide, binds to the immobilized polypeptide. The second antibody may be labelled by methods known in the art. Non-immobilized materials are washed away, and the presence of immobilized label indicates the presence of the polypeptide. This and other immunoassays are described by David, et al., in U.S. Patent 4,376,110 assigned to Hybritech, Inc., La Jolla, California.

The probe may also be a nucleic acid molecule that recognizes a RNA or DNA molecule that encodes a member of the hemolysin family of toxins present in N. meningitidis. Methods for determining whether a nucleic acid molecule probe recognizes a specific nucleic acid molecule in a sample are known in the art. Generally, a labelled probe that is complementary to a nucleic acid sequence suspected of being in a sample is prepared. The presence of probe hybridized to the target nucleic acid molecule indicates the presence of the nucleic acid molecule. Suitable methods are described by Schneider et al in U.S. Patent 4,882,269, which is assigned to Princeton University, and by Segev in PCT Application WO 90/01069. The Schneider et al patent and the Segev application are both licensed to ImClone Systems Inc., New York City.

The probes described above are labelled in accordance with methods known in the art. Methods for labelling antibodies have been described, for example, by Hunter and Greenwood in Nature 144, 945 (1962) and by David et al in Biochemistry 13, 1014-1021 (1974). Additional methods for labelling antibodies have been described in U.S. patents 3,940,475 and 3,645,090. Methods for labelling oligonucleotide probes have been described, for example, by Leary et al, Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al, Nucl. Acids Res. (1985) 13:2399; and Meinkoth and Wahl, Anal. Biochem. (1984) 138:267.

The label may be radioactive. Some examples of useful radioactive labels include ³²P,¹²⁵I, ¹³¹I, and ³H. Use of radioactive labels have been described in U.K. 2,034,323, U.S. 4,358,535, and U.S. 4,302,204.

Some examples of non-radioactive labels include enzymes, chromophors, atoms and molecules detectable by electron microscopy, and metal ions detectable by their magnetic properties.

Some useful enzymatic labels include enzymes that cause a detectable change in a substrate. Some useful enzymes and their substrates include, for example, horseradish peroxidase (pyrogallol and o-phenylenediamine), beta-galactosidase (fluorescein beta-D-galactopyranoside), and alkaline phosphatase (5-bromo-4-chloro-3-indolyl phosphate/nitro blue tetrazolium). The use of enzymatic labels have been described in U.K. 2,019,404, EP 63,879, and by Rotman, Proc. Natl. Acad. Sci., 47, 1981-1991 (1961).

Useful chromophores include, for example, fluorescent, chemiluminescent, and bioluminescent molecules, as well as dyes. Some specific chromophores useful in the present invention include, for example, fluorescein, rhodamine, Texas red, phycoerythrin, umbelliferone, luminol.

The labels may be conjugated to the antibody or nucleotide probe by methods that are well known in the art. The labels may be directly attached through a functional group on the probe. The probe either contains or can be caused to contain such a functional group. Some examples of suitable functional groups include, for example, amino, carboxyl, sulfhydryl, maleimide, isocyanate, isothiocyanate.

The label may also be conjugated to the probe by means of a ligand attached to the probe by a method described above and a receptor for that ligand attached to the label. Any of the known ligand-receptor combinations is suitable. The biotin-avidin combination is preferred.

The polypeptide of the invention may be used to detect the presence of antibodies specific for N. meningitidis in a sample. The method comprises preparing a polypeptide containing a segment having an amino acid sequence that is substantially homologous to a member of the hemolysin family of toxins. The polypeptide may be prepared as described above. Preferably, the polypeptide comprises a segment having an amino acid sequence that is present in N. meningitidis.

The sample may, for example, be from a patient suspected of being infected with N. meninaitidis. Suitable assays are known in the art, such as the standard ELISA protocol described by R.H. Kenneth, "Enzyme-Linked Antibody Assay with Cells Attached to Polyvinyl Chloride Plates" in Kenneth et al, Monoclonal Antibodies, Plenum Press, N.Y., page 376 (1981).

Briefly, plates are coated with antigenic polypeptide at a concentration sufficient to bind detectable amounts of the antibody. After incubating the plates with the polypeptide, the plates are blocked with a suitable blocking agent, such as, for example, 10% normal goat serum. The sample, such as patient sera, is added and titered to determine the endpoint. Positive and negative controls are added simultaneously to quantitate the amount of relevant antibody present in the unknown samples. Following incubation, the samples are probed with goat anti-human Ig conjugated to a suitable enzyme. The presence of anti-polypeptide antibodies in the sample is indicated by the presence of the enzyme.

Antibodies raised against polypeptides of the present invention are capable of recognizing N. meningitidis and distinguishing meningococcal cells from gonococcal cells in a sample. The A4.85 monoclonal antibody, for example, recognizes a polypeptide expressed by iron-stressed meningococcal cells. A4.85 does not, however, recognize any proteins in iron-stressed N. gonorrhoeae.

The antibodies may be labelled by known methods as described above. Assays for distinguishing iron-stressed meningococcal cells from iron-stressed gonococcal cells follow known formats, such as standard blot and ELISA formats.

### EXAMPLES

### Example 1. Isolation of A4.85 MAb

Bacterial outer membranes are prepared from iron-stressed cultures of Neisseria meningitidis strain FAM20 as follows. FAM20 is inoculated into chelexed defined medium (CDM, West et al, J. Bacteriology 169, 3414 (1987)). This medium allows growth only until iron stores within the bacteria have been depleted. During this time, a set of proteins that are regulated by the availability of iron become expressed. Bacteria are harvested and Outer membranes are prepared as described by Dyer et al in Infection and Immunity 56, 977 (1988).

Three to five BALB/c female mice are immunized with iron-stressed FAM20 outer membranes by either the intramuscular (im) or intraperitoneal (ip) routes. With the im route, 100 µg of antigen (Ag) is emulsified in complete Freund's adjuvant and injected on two different sites on day zero, followed by booster doses two weeks apart with the Ag now emulsified in incomplete Freund's adjuvant. The ip route involves immunization with 100 µg of Ag on days zero, 7, 14 and 28. Serum antibody levels are checked by either ELISA or Western blotting three days following the final boost to determine serum antibody levels. Mice are given a final boost ip on each of three consecutive days before the fusion. On the day of the fusion, mice are sacrificed by cervical dislocation and the spleens are removed aseptically. Spleen cells are extracted by teasing the cells out of the sac using two bent 19 ga needles. Extracted cells are resuspended to give single cell suspensions.

Mouse myeloma cells, SP2.0-AG14 (ATCC CRL 1581), that have been grown in Dulbecco's Modified Eagle's Medium/Ham's F-12 (DMEM/F12) supplemented with 15% .fetal calf serum (FCS), are used as the fusion partner. Cells are mixed in a 10:1 ratio of spleen:myeloma cells and pelleted together in a 50 ml centrifuge tube. The supernatant is aspirated off leaving a dry pellet to which 1 ml of 50% polyethylene glycol (PEG) (prewarmed to 37°C) is added. The cells are gently resuspended and allowed to sit at room temperature for 2 minutes, after which 1 ml of DMEM/F12 without added sera is added and the cells gently resuspended. The cells are then further diluted and resuspehded with 2, 4, 8 and 16 ml of DMEM/F12 added at two-minute intervals. The cells are then pelleted and the supernatant aspirated. Two ml of DMEM/F12 supplemented with 15% FCS is carefully added to avoid resuspension of the pellet and then incubated for one hour at 37°C.

At the end of the 1 hour incubation, the suspension is diluted to a final concentration of 1x10⁶ cells/ml. This suspension is then poured into tissue culture flasks and incubated overnight. The next day an equal volume of culture media supplemented with 2X HAT (hypoxanthine, aminopterin, thymidine) components are plated out into 96-well plates with 200 µl/well with 1x10⁵ spleen cells/well. Plated calls are fed every 4-5 days after the fusion by aspiration of half the media from the wells and addition of fresh 1xHAT media. The wells are scored for growth after 10-14 days, and growing wells are tested for presence of secreted antibody by screening the culture supernatants by either an ELISA or Western blotting. Wells that prove positive on assay are expanded for growth into 24-well culture dishes in culture media with HT supplements (no aminopterin) and re-tested. Those proving positive on re-testing are expanded further into larger tissue culture vessels and then cloned twice by limiting dilution.

A cell line (A4.85) that arose from a single mouse spleen cell was isolated. A4.85 produces a monoclonal antibody (MAb) that reacts with several protein species (70 kilodaltons to several hundred kilodaltons in mass) on a Western blot of FAM20 outer membranes, each of whose synthesis is repressed by the presence of iron in the bacterial growth medium.

### Example 2A. Isolation of Genomic Clones

### A. Library construction

A library of Neisseria meningitidis strain FAM20 chromosomal DNA is constructed in the bacteriophage vector lambda-gt11 as follows. FAM20 chromosomal DNA is isolated by standard methods (Maniatis et al, 1982). The DNA is sheared by sonication to fragment sizes of approximately 300-1000 bp. Synthetic EcoRI linkers are ligated to the ends of these molecules, followed by cleavage with EcoRI restriction endonuclease to generate EcoRI restriction sites at the end of each molecule. The resulting fragments are ligated with EcoRI-cleaved lambda-gt11 DNA (Maniatis et al, Molecular Cloning: A Laboratory Manual, Cold Spring Harbor Laboratory Press, Cold Spring Harbor, New York (1982)). The ligated DNA is packaged into lambda phage heads using lambda packaging extracts (Promega Corp., Madison, Wisconsin), according to manufacturer's instructions.

### B. Library Screening and Isolation of DNA

The library created above is screened with the A4.85 MAb to detect clones that express the epitope recognized by A4.85. 500,000 recombinant plaques from the lambda-gt11 expression library are screened by the method of Maniatis et al (1982). A pure clone reacting with the A4.85 MAb is isolated by re-plating and screening the reactive plaque twice. The meningococcal insert DNA from the pure lambda clone (lambda 4.85) is amplified by the polymerase chain reaction (PCR) technique using a kit from Perkin-Elmer/Cetus. The PCR-amplified DNA is cloned into the sequencing vector M13mp19 (Maniatis et al, 1982) and the DNA sequence determined by the dideoxy chain termination method of Sanger et al (Proc. Natl. Acad. Sci. USA 74, 5463-5467 (19877)) using the Sequenase kit (Stratagene, La Jolla, CA).

The cloned meningococcal DNA is labelled with ³²Pby the random primed method with a kit from Boehringer-Mannheim (Indianapolis, IN) and is used in Southern hybridizations (Maniatis et al, 1982) to identify DNA restriction fragments in the FAM20 chromosome adjacent to the DNA cloned in lambda 4.85. Chromosomal Sau3A I fragments of approximately 560 and 1600 bp hybridize to the cloned meningococcal DNA. FAM20 DNA is cleaved with Sau3A I and fractionated on a preparative agarose gel. Two size fractions are isolated, one of 400-700 bp and one of 1400-1800 bp.

The 560 bp Sau3A I fragment is cloned by ligating the 400-700 bp fraction of FAM20-Sau3A I fragments with BamHI-cleaved plasmid pBR322 (Maniatis et al, 1982). The desired clones of the 560 bp fragment are identified by hybridization of bacterial colonies containing recombinant plasmids with ³²P-labelledlambda 4.85 insert DNA (Maniatis et al, 1982). Plasmid DNA (pUNCH201) from a pure colony hybridizing with the DNA probe is prepared and its sequence determined using Sequenase® as modified for use in double-strand sequencing (Kraft et al, BioTechniques 6, 544 (1988)). Southern hybridization is used to verify that the cloned fragment is representative of the fragment intact in the FAM20 genome.

To clone the 1600 bp fragment, the ends of the 1400-1800 bp fraction of FAM20-Sau3A I fragments are made blunt by reaction with Klenow enzyme and DNA nucleotides. Synthetic EcoRI linkers are added to these molecules, followed by ligation with EcoRI-cleaved, alkaline-phosphatase-treated lambda ZAP DNA (Stratagene, LaJolla, CA) in accordance with technical information supplied with the lambda ZAP kit. Ligated DNA is packaged into lambda heads using Packagene® lambda packaging extracts (Promega). The library of 1400-1800 bp FAM20-Sau3A I fragments is screened with a ³²P-labelledoligonucleotide (SAT1), which is synthesized to correspond to DNA sequences at one end of the lambda 4.85 insert (5' GCCATTGCCACTGTAGATA 3'). A lambda ZAP® plaque hybridizing with the SAT1 oligonucleotide is purified as above. The interior portion of this lambda ZAP® clone (lambda ZAP202®) is "excised" by the addition of helper bacteriophage. The excision results .in a multicopy plasmid (pUNCH202) containing the cloned meningococcal insert. Southern hybridization is used to verify that the cloned fragment is representative of the fragment intact in the FAM20 genome. The sequence of the cloned DNA fragment is determined by double-strand sequencing as described above.

The adjoined sequence of pUNCH201, pUNCH202, and the lambda 4.85 insert reveals the presence of an open reading frame that contains the entirety of the cloned DNA. Neither the start or end of the gene is present within this cloned DNA. The DNA sequence is shown as Figure 1. The amino acid sequence predicted by the open reading frame contains 835 amino acids (91kD). The sequence is shown as Figure 2.

As determined by FASTA sequence comparison searches (see above), both the DNA and the deduced polypeptide sequence from this region have a high degree of similarity with a family of hemolysin bacterial toxins. For example, the DNA sequence shown in Figure I exhibits 54% identity with the cya gene (adenylate cyclase) from B. pertussis; 60% identity with the hlyA, hlyB, hlyC and hlyD gene from E. coli (hemolysin); 65% identity with hlyA, hlyB and hlyC gene (hemolysin) from E. coli; 56% identity with the leukotoxin gene from A. actinomycetemcomitans; 56% identity with the hemolysin gene from A. pleuropneumoniae; 60% identity with the leukotoxin gene from P. haemolytica; 62% identity with the A1 leukotoxin gene from P. haemolytica; and 57% identity with protease B gene of E. chrysanthemi.

The amino acid sequence predicted from the DNA sequence exhibited 25%-28% identity with leukotoxin, 22%-28% identity with hemolysin; and 30% identity with adenylate cyclase.

Meningococcal strain FAM20 contains at least two copies of DNA that encode the polypeptides of the invention. This can be demonstrated by digesting genomic DNA with the infrequent cutters BglII, Spel, NheI, and combinations of NheI and SpeI. Southern blots of the digested DNA separated by pulse field gradient electrophoresis reveal two major bands that hybridize under stringent conditions to gene probes containing fragments of the sequence of the gene that encodes the polypeptide of the invention.

The remainder of the gene encoding the iron-regulated polypeptide of the invention is isolated in a manner similar to that described above for isolating pUNCH201 and pUNCH202. DNA restriction fragments either flanking the ends of the region already cloned or containing the entire region are identified by Southern hybridization using oligonucleotide probes derived from previously determined DNA sequence. These fragments are cloned into either plasmid or bacteriophage vectors as described above for pUNCH201 and pUNCH202. The DNA sequence of newly cloned fragments is determined as above, and reveals when either end of the gene is reached. If the gene is isolated on a single DNA fragment, it is expressed in an in vitro assay to verify that the protein that is encoded by this gene reacts with the A4.85 MAb. If the gene is not cloned intact on a single DNA fragment, it is reconstructed through standard molecular biology techniques to yield the intact gene (Carbonetti, Proc. Natl. Acad. Sci. USA 84, 9084 (1987)).

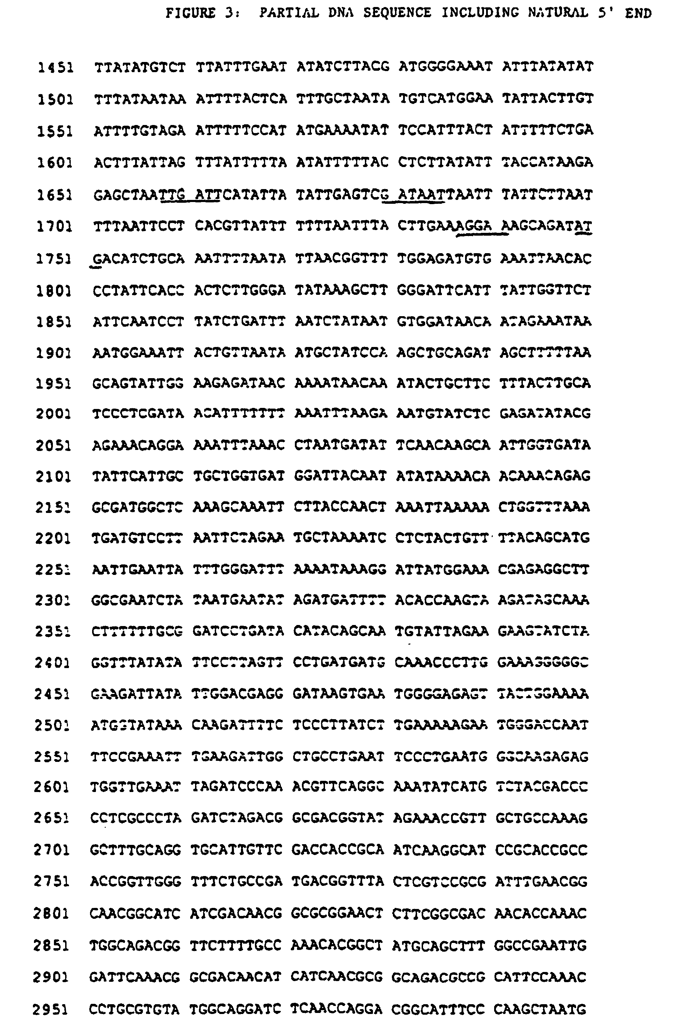

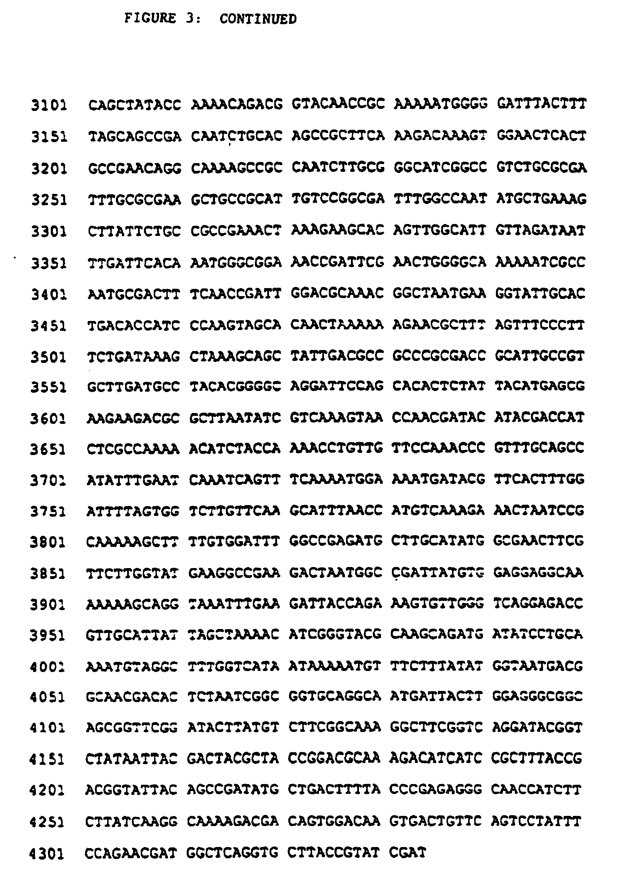

For example, DNA fragments from one of the two copies of the structural genes coding for the polypeptide of the invention were purified from agarose gels, cloned and sequenced. Figure 3 shows the DNA sequence, which is complete in the 5' end of the gene. Underlined in Figure 3 are a typical promoter with a -35 and -10 region, a ribosome binding site, an ATG start site, and a consensus fur box, which is typically found in many gram negative iron-regulated promoters.

### Example 2B. Western Blot and Molecular Weight

The full length polypeptide obtained from meningococcal strain FAM20 exhibits a molecular weight of 230-250 kD when subjected to Western blot analysis. Western blots may be carried out as follows:

Iron-starved whole cells of FAM20 are prepared in accordance with the method of West and Sparling, J. Bacteriol. 169, 3414-3421 (1987). The cells are washed in ice-cold Davis Minimal Medium A (Lederberg, Methods in Med. Res., 3:5 (1950)), immediately cooled on ice, and ruptured in a French pressure cell at 0°C and 20,000 psi.

The resulting mixture is centrifuged for 10 minutes at 20,000G, and the pellet solubilized in boiling SDS. The solubilized membrane proteins are separated by standard 7.5% SDS-PAGE in Laemmli buffer, which was described by Laemmli in Nature 227, 680-685 (1970). The proteins are transferred (16 hours, 80 uA) onto Optibind® nitrocellulose membranes (available from from Schleicher & Schuell). The membranes are blocked for 1 hour in 5% BSA in TBS (20mM Tris, 500 mM NaCl, pH 7.5); rinsed for 5 minutes in TBS; incubated for 1 hour with 1:2 dilution of monoclonal antibody A4.85 (see above) in 5% BSA; washed twice for 5 minutes in TBS and 0.05% Tween 20; incubated for 1 hour in a secondary antibody (goat anti-mouse lgG alkaline phosphatase conjugate) diluted in 5% BSA, available from BioRad (dilution = 1:3000) or Sigma (dilution = 1:1000); washed twice for 5 minutes in TBS/Tween; washed again for 5 minutes in TBS; and developed with an alkaline phosphatase substrate comprising 45ul Nitro Blue Tetrazolin, available from Sigma (75 mg/ml); 35 ul 5-bromo-4-chloro-3-indolylphosphate, p-tolnidine salt (So mg/ml) in 10 ml of carbonate buffer, pH 9.8 (0.1 M NaHCO₃; 1mM MgCl₂).

### Example 3. Assay for Antibody in Sample

A standard ELISA protocol is used to screen for the presence of antibodies against the polypeptide in proteins. Briefly, 96 well microtiter plates are coated with the antigen at concentrations varying from 50-1000ng per well in a high ph (9.6) carbonate buffer. The plates are incubated overnight at 9°C and blocked with 10% normal goat serum for one hour at 37°C. Patient sera is added and titered to determine the endpoint. Control positive and negative sera is added at the same time to quantitate the amount of relevant antibody present in the unknown samples. After a 2-3 hour incubation at 37°C, samples are probed with goat anti-human Ig conjugated to horseradish peroxidase. Positive samples are determined by using TMB.

The invention as claimed is enabled in accordance with the specification and readily available references and starting materials. Nevertheless, the following cell lines have been deposited in the American Type Culture Collection, Bethesda, Maryland on July 12, 1990 in order to facilitate the making and using of the invention:
Meningococcal cell line FAM18 (Accession Number 55071)
Meningococcal cell line FAM20 (Accession Number 55072)
Hybridoma cell line A4.85 (Accession Number HB 10504)

In addition, the following brochures containing useful protocols and information are available in the file history of this specification.
"Predigested Lambda Zap/Eco RI Cloning Kit Instruction Manual," Stratagene, La Jolla, California (November 20, 1987);
"Gigapack Plus" (for packaging recombinant lambda phage), Stratagene, La Jolla, California (April 25, 1988); and
"picoBlue Immunoscreening Kit" Instruction Manual," Stratagene, La Jolla, California (May 19, 1989).

## Claims

1. An essentially pure, antigenic polypeptide having at least 900 amino acids comprising a segment of at least fifty amino acid residues and having an amino acid sequence present in *N. meningitidis*, wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

2. The polypeptide of claim 1, wherein the segment has at least 100 amino acid residues.

3. The polypeptide of claim 1, wherein the segment has at least 200 amino acid residues.

4. The polypeptide of claim 1, wherein the antigenic amino acid sequence is immunogenic.

5. The polypeptide of claim 1, wherein the amino acid sequence of the polypeptide comprises the sequence shown in Figure 2.

6. The polypeptide of claim 1, wherein antibodies against the polypeptide cross-react with at least one other member of the hemolysin family of toxins from other genera of bacteria.

7. The polypeptide of claim 6, wherein the other genera of bacteria include *Escherichia, Serratia, Pasteurella, Proteus, Actinobacillus,* and *Bordetella.*

8. The polypeptide of claim 6, wherein the other members of the hemolysin family of toxins comprise alpha-hemolysin from *Escherichia coli*; leukotoxin from *Actinobacillus actinomycetemcomitans*; leukotoxin from *Pasteurella haemolytica;* adenylate cyclase from *Bordetella pertussis* and adenylate cyclase from *Bacillus anthracis*.

9. The polypeptide of claim 6, wherein the other member of the hemolysin family of toxins is alpha-hemolysin from *E. coli.*

10. A method of producing an antigen useful in protecting a mammal from infection by *N. meningitidis* comprising the steps of:
(a) preparing an essentially pure, antigenic polypeptide having at least 900 amino acids comprising a segment having at least fifty amino acid residues, wherein the amino acid sequence is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue.
(b) rendering the polypeptide non-toxic to mammals.

11. The method of claim 10, wherein the amino acid sequence is present in a polypeptide found in *N. meningitidis.*

12. The method of claim 10, wherein the mammal is a human.

13. A method of producing a vaccine composition useful in protecting a mammal from infection by *N. meningitidis* comprising the steps of:
(a) preparing an essentially pure, antigenic polypeptide having at least 900 amino acids comprising a segment having at least fifty amino acid residues, wherein the amino acid sequence is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue;
(b) rendering the polypeptide non-toxic to mammals; and
(c) combining the non-toxic polypeptide with a pharmaceutically acceptable carrier.

14. The method of claim 13, wherein the polypeptide is isolated from *N. meningitidis.*

15. The method of claim 13, wherein a the mammal is a human.

16. A vaccine composition capable of immunizing mammals against infections by *N. meningitidis*, the vaccine composition comprising:
(a) an immunogenic polypeptide having at least 900 amino acids that is non-toxic to mammals and comprises a segment having at least fifty amino acid residues, wherein the amino acid sequence is different from, but substantially homologous with, the hemolysin family of toxins, or antigenic fragments of such polypeptides, wherein the antigenic fragments comprise at least three repeats of the hemolysin consensus sequence LXGGXGNDX, wherein X, independently, represents any single amino acid residue, and
(b) a pharmaceutically acceptable carrier.

17. The vaccine composition of claim 16, wherein the polypeptide is present in *N. meningitidis*.

18. The vaccine composition of claim 17, wherein the polypeptide is present in outer membranes of *N. meningitidis*.

19. The vaccine composition of claim 16, wherein the mammal is a human.

20. Monoclonal antibodies raised against a polypeptide having at least 900 amino acids comprising a segment of at least fifty amino acid residues and having an amino acid sequence present in *N. meningitidis*, wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

21. An isolated nucleic acid molecule that encodes a polypeptide having at least 900 amino acids comprising a segment of at least fifty amino acid residues and having an amino acid sequence present in *N. meningitidis*, wherein the amino acid sequence of the segment is different from, but substantially homologous with the amino acid sequence of a segment of a member of the hemolysin family of toxins, and antigenic fragments of such polypeptides, wherein the antigenic fragments comprise at least three repeats of the hemolysin consensus sequence LXGGXGNDX, wherein X, independently, represents any single amino acid residue.

22. A method of detecting the presence of antibodies specific for a member of the hemolysin family of toxins in a sample comprising the steps of:
(a) preparing an essentially pure, antigenic polypeptide having at least 900 amino acids comprising a segment having at least fifty amino acid residues and wherein the polypeptide comprises an amino acid sequence present in *N. meningitidis*, wherein the amino acid sequence of the segment is different from, but substantially homologous with, the amino acid sequence of a segment of a member of the hemolysin family of toxins, or antigenic fragments of such polypeptides, wherein the antigenic fragments comprise at least three repeats of the hemolysin consensus sequence LXGGXGNDX, wherein X, independently, represents any single amino acid residue; and
(b) determining whether the polypeptide recognizes an antibody in the sample.

23. An essentially pure polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, and antigenic fragments of such polypeptides, wherein the antigenic fragments comprise at least three repeats of the hemolysin consensus sequence LXGGXGNDX, wherein X, independently, represents any single amino acid residue.

24. The polypeptide of claim 23, wherein the segment has at least 100 amino acid residues.

25. The polypeptide of claim 23, wherein the segment has at least 200 amino acid residues.

26. The polypeptide of claim 23, wherein the antigenic amino acid sequence is immunogenic.

27. The polypeptide of claim 23, wherein antibodies against the polypeptide cross-react with at least one other member of the hemolysin family of toxins from other genera of bacteria.

28. The polypeptide of claim 23, wherein the other genera of bacteria include *Escherichia*, *Serratia*, *Pasteurella*, *Proteus*, *Actinobacillus*, and *Bordetella*.

29. The polypeptide of claim 27, wherein the other members of the hemolysin family of toxins comprise alpha-hemolysin from *Escherichia coli*, leukotoxin from *Actinobacillus actinomycetemcomitans*, leukotoxin from *Pasteurella haemolytica*, adenylate cyclase from *Bordetella pertussis* and adenylate cyclase from *Bacillus anthracis.*

30. The polypeptide of claim 27, wherein the other member of the hemolysin family of toxins is alpha-hemolysin from *E. coli.*

31. A method of producing an antigen useful in protecting a mammal from infection by *N. meningitidis* comprising the steps of:
(a) preparing an essentially pure polypeptide having at least 900 amino acids comprising a segment having an amino acid sequence that consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue; and
(b) rendering the polypeptide or fragment non-toxic to mammals.

32. The method of claim 31, wherein the amino acid sequence is present in a polypeptide found in *N. meningitidis*.

33. A method of producing a vaccine composition useful in protecting a mammal from infection by *N. meningitidis* comprising the steps of:
(a) preparing an essentially pure, antigenic polypeptide having at least 900 amino acids comprising a segment having an amino acid sequence that consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, or antigenic fragments of such polypeptides, wherein the antigenic fragments comprise at least three repeats of the hemolysin consensus sequence LXGGXGNDX, wherein X independently, represents any single amino acid residue, and
(b) rendering the polypeptide non-toxic to mammals; and
(c) combining the non-toxic polypeptide with a pharmaceutically acceptable carrier.

34. A method according to claim 33, wherein the polypeptide is isolated from *N. meningitidis.*

35. A vaccine composition capable of immunizing mammals against infections by *N. meningitidis*, the vaccine composition comprising:
(a) an essentially pure, immunogenic polypeptide having at least 900 amino acids comprising a segment having an amino acid sequence that consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue, and
(b) a pharmaceutically acceptable carrier.

36. The vaccine compostion of claim 35, wherein the polypeptide is present in *N. meningitidis*.

37. The vaccine composition of claim 36 wherein the polypeptide is present in outer membranes of *N. meningitidis.*

38. The vaccine composition of claim 35 wherein the mammal is a human.

39. Monoclonal antibodies raised against an essentially pure polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue.

40. An isolated nucleic acid molecule that encodes a polypeptide having at least 900 amino acids comprising a segment having an amino acid sequence present in *N. meningitidis*, wherein the amino acid sequence consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue, or antigenic fragments of such polypeptides, wherein the antigenic fragments comprise at least three repeats of the hemolysin consensus sequence LXGGXGNDX, wherein X, independently, represents any single amino acid residue.

41. A method of detecting the presence of *N. meningitidis* in a sample comprising the steps of:
(a) preparing a probe that recognizes a polypeptide that is a member of the hemolysin family of toxins or a fragment thereof, or a nucleic acid molecule encoding the polypeptide or fragment wherein the polypeptide that is a member of the hemolysin family of toxins or nucleic acid molecule encoding the polypeptide is present in *N. meningitidis*; and
(b) determining whether the probe recognizes *N. meningitidis* in the sample.

42. The method of claim 41, wherein the probe is an antibody.

43. The method of claim 42, wherein the antibody is monoclonal.

44. The method of claim 41, wherein the probe is a nucleic acid molecule.

45. A method of detecting the presence of antibodies specific for a member of the hemolysin family of toxins in a sample comprising the steps of:
(a) preparing an antigenic polypeptide having at least 900 amino acids and wherein the polypeptide comprises an amino acid sequence present in *N. meningitidis* comprising a segment having an amino acid sequence that consists of at least three repeats of the nine amino acid hemolysin consensus sequence, the hemolysin consensus sequence consisting of at least four of:
L at position 1;
G at position 3;
G at position 4;
G at position 6;
N at position 7;
D at position 8; and
x at positions 2, 5 and 9;
wherein x represents any single amino acid residue; and
(b) determining whether the polypeptide recognizes an antibody in the sample, or antigenic fragments of such polypeptides, wherein the antigenic fragments comprise at least three repeats of the hemolysin consensus sequence LXGGXGNDX, wherein X, independently, represents any single amino acid residue.

## Patentansprüche

1. Ein im wesentlichen reines, antigenes Polypeptid, das mindestens 900 Aminosäuren besitzt, umfassend ein Segment von mindestens fünfzig Aminosäureresten, und das eine Aminosäuresequenz besitzt, die in *N. meningitidis* vorhanden ist, wobei die Aminosäuresequenz des Segmentes unterschiedlich von aber im wesentlichen homolog mit der Aminosäuresequenz eines Segmentes eines Mitgliedes der Hämolysin-Familie der Toxine ist, und antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert.

2. Das Polypeptid gemäß Anspruch 1, wobei das Segment mindestens 100 Aminosäurereste besitzt.

3. Das Polypeptid gemäß Anspruch 1, wobei das Segment mindestens 200 Aminosäurereste besitzt.

4. Das Polypeptid gemäß Anspruch 1, wobei die antigene Aminosäuresequenz immunogen ist.

5. Das Polypeptid gemäß Anspruch 1, wobei die Aminosäuresequenz des Polypeptids die in Figur 2 gezeigte Sequenz umfaßt.

6. Das Polypeptid gemäß Anspruch 1, wobei Antikörper gegen das Polypeptid mit mindestens einem anderen Mitglied der Hämolysin-Familie der Toxine aus einer anderen Gattung von Bakterien eine Kreuzreaktion eingehen.

7. Das Polypeptid gemäß Anspruch 6, wobei die anderen Gattungen von Bakterien *Escherichia*, *Serratia*, *Pasteurella*, *Proteus, Actinobacillus* und *Bordetella* mit einschließen.

8. Das Polypeptid gemäß Anspruch 6, wobei die anderen Mitglieder der Hämolysin-Familie der Toxine alpha-Hämolysin aus *Escherichia coli*, Leukotoxin aus *Actinobacillus actinomycetemcomitans*, Leukotoxin aus *Pasteurella haemolytica*, Adenylatzyklase aus *Bordetella pertussis* und Adenylatzyklase aus *Bacillus anthracis* mit einschließen.

9. Das Polypeptid gemäß Anspruch 6, wobei das andere Mitglied der Hämolysin-Familie der Toxine alpha-Hämolysin aus *E. coli* ist.

10. Ein Verfahren zur Herstellung eines Antigens, das zum Schutz eines Säugetiers vor einer Infektion durch *N. meningitidis* nützlich ist, und das die Schritte umfaßt:
(a) Herstellen eines im wesentlichen reinen, antigenen Polypeptids, das mindestens 900 Aminosäuren besitzt, umfassend ein Segment das mindestens fünfzig Aminosäurereste besitzt, wobei die Aminosäuresequenz unterschiedlich von aber im wesentlichen homolog mit der Aminosäuresequenz eines Segmentes eines Mitgliedes der Hämolysin-Familie der Toxine ist, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert;
(b) das Polypeptid nicht-toxisch für Säugetiere machen.

11. Das Verfahren gemäß Anspruch 10, wobei die Aminosäuresequenz in einem Polypeptid vorhanden ist, das in *N. meningitidis* gefunden wird.

12. Das Verfahren gemäß Anspruch 10, wobei das Säugetier ein Mensch ist.

13. Ein Verfahren zur Herstellung einer Vakzinzusammensetzung, die zum Schutz eines Säugetiers vor einer Infektion durch *N. meningitidis* nützlich ist, und das die Schritte umfaßt:
(a) Herstellen eines im wesentlichen reinen, antigenen Polypeptids, das mindestens 900 Aminosäuren besitzt, umfassend ein Segment das mindestens fünfzig Aminosäurereste besitzt, wobei die Aminosäuresequenz unterschiedlich von aber im wesentlichen homolog mit der Aminosäuresequenz eines Segmentes eines Mitgliedes der Hämolysin-Familie der Toxine ist, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert;
(b) das Polypeptid nicht-toxisch für Säugetiere machen; und
(c) Kombinieren des nicht-toxischen Polypeptides mit einem pharmazeutisch geeigneten Träger.

14. Das Verfahren gemäß Anspruch 13, wobei das Polypeptid aus *N. meningitidis* isoliert wird.

15. Das Verfahren gemäß Anspruch 13, wobei das Säugetier ein Mensch ist.

16. Eine Vakzinzusammensetzung, die zur Immunisierung von Säugetieren gegen Infektionen durch *N. meningitidis* fähig ist, wobei die Vakzinzusammensetzung umfaßt:
(a) ein immunogenes Polypeptid, das mindestens 900 Aminosäuren besitzt, welches nicht-toxisch für Säugetiere ist, und das ein Segment umfaßt, das mindestens fünfzig Aminosäurereste besitzt, wobei die Aminosäuresequenz unterschiedlich von aber im wesentlichen homolog mit einem Mitglied der Hämolysin-Familie der Toxine ist, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert; und
(b) einen pharmazeutisch geeigneten Träger.

17. Die Vakzinzusammensetzung gemäß Anspruch 16, wobei das Polypeptid in *N. meningitidis* vorhanden ist.

18. Die Vakzinzusammensetzung gemäß Anspruch 17, wobei das Polypeptid in den äußeren Membranen von *N. meningitidis* vorhanden ist.

19. Die Vakzinzusammensetzung gemäß Anspruch 16, wobei das Säugetier ein Mensch ist.

20. Monoklonale Antikörper erzeugt gegen ein Polypeptid, das mindestens 900 Aminosäuren besitzt, umfassend ein Segment von mindestens fünfzig Aminosäureresten, und das eine Aminosäuresequenz besitzt, die in *N. meningitidis* vorhanden ist, wobei die Aminosäuresequenz des Segmentes unterschiedlich von aber im wesentlichen homolog mit der Aminosäuresequenz eines Segmentes eines Mitgliedes der Hämolysin-Familie der Toxine ist, und antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert.

21. Ein isoliertes Nukleinsäuremolekül, das ein Polypeptid kodiert, das mindestens 900 Aminosäuren besitzt, umfassend ein Segment von mindestens fünfzig Aminosäureresten, und das eine Aminosäuresequenz besitzt, die in *N. meningitidis* vorhanden ist, wobei die Aminosäuresequenz des Segmentes unterschiedlich von aber im wesentlichen homolog mit der Aminosäuresequenz eines Segmentes eines Mitgliedes der Hämolysin-Familie der Toxine ist, und antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert.

22. Ein Verfahren zum Detektieren der Anwesenheit von Antikörpern, die spezifisch für ein Mitglied der Hämolysin-Familie der Toxine sind, in einer Probe, und wobei das Verfahren die Schritte umfaßt:
(a) Herstellen eines im wesentlichen reinen, antigenen Polypeptids, das mindestens 900 Aminosäuren besitzt, umfassend ein Segment das mindestens fünfzig Aminosäurereste besitzt, und wobei das Polypeptid eine Aminosäuresequenz umfaßt, die in *N. meningitidis* vorhanden ist, wobei die Aminosäuresequenz des Segmentes unterschiedlich von aber im wesentlichen homolog mit der Aminosäuresequenz eines Segmentes eines Mitgliedes der Hämolysin-Familie der Toxine ist, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert;
(b) Bestimmen, ob das Polypeptid einen Antikörper in der Probe erkennt.

23. Ein im wesentlichen reines Polypeptid, das mindestens 900 Aminosäuren besitzt, umfassend ein Segment, das eine Aminosäuresequenz besitzt, die in *N. meningitidis* vorhanden ist, wobei die Aminosäuresequenz aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, und antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert;

24. Das Polypeptid gemäß Anspruch 23, wobei das Segment mindestens 100 Aminosäurereste besitzt.

25. Das Polypeptid gemäß Anspruch 23, wobei das Segment mindestens 200 Aminosäurereste besitzt.

26. Das Polypeptid gemäß Anspruch 23, wobei die antigene Aminosäuresequenz immunogen ist.

27. Das Polypeptid gemäß Anspruch 23, wobei Antikörper gegen das Polypeptid mit mindestens einem anderen Mitglied der Hämolysin-Familie der Toxine aus einer anderen Gattung von Bakterien eine Kreuzreaktion eingehen.

28. Das Polypeptid gemäß Anspruch 23, wobei die anderen Gattungen von Bakterien *Escherichia*, *Serratia*, *Pasteurella*, *Proteus, Actinobacillus* und *Bordetella* mit einschließen.

29. Das Polypeptid gemäß Anspruch 27, wobei die anderen Mitglieder der Hämolysin-Familie der Toxine alpha-Hämolysin aus *Escherichia coli*, Leukotoxin aus *Actinobacillus actinomycetemcomitans*, Leukotoxin aus *Pasteurella haemolytica*, Adenylatzyklase aus *Bordetella pertussis* und Adenylatzyklase aus *Bacillus anthracis* mit einschließen.

30. Das Polypeptid gemäß Anspruch 27, wobei das andere Mitglied der Hämolysin-Familie der Toxine alpha-Hämolysin aus *E. coli* ist.

31. Ein Verfahren zur Herstellung eines Antigens, das zum Schutz eines Säugetieres vor einer Infektion durch *N. meningitidis* nützlich ist, und das die Schritte umfaßt:
(a) Herstellen eines im wesentlichen reinen Polypeptids, das mindestens 900 Aminosäuren besitzt, umfassend ein Segment, das eine Aminosäuresequenz besitzt, die aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert; und
(b) das Polypeptid oder ein Fragment nicht-toxisch für Säugetiere machen.

32. Das Verfahren gemäß Anspruch 31, wobei die Aminosäuresequenz in einem Polypeptid vorhanden ist, das in *N. meningitidis* gefunden wird.

33. Ein Verfahren zur Herstellung einer Vakzinzusammensetzung, die zum Schutz eines Säugetieres vor einer Infektion durch *N. meningitidis* nützlich ist, und das die Schritte umfaßt:
(a) Herstellen eines im wesentlichen reinen, antigenen Polypeptids, das mindestens 900 Aminosäuren besitzt, umfassend ein Segment, das eine Aminosäuresequenz besitzt, die aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert; und
(b) das Polypeptid nicht-toxisch für Säugetiere machen; und
(c) Kombinieren des nicht-toxischen Polypeptides mit einem pharmazeutisch geeigneten Träger.

34. Das Verfahren gemäß Anspruch 33, wobei das Polypeptid aus *N. meningitidis* isoliert wird.

35. Eine Vakzinzusammensetzung, die zur Immunisierung von Säugetieren gegen Infektionen durch *N. meningitidis* fähig ist, wobei die Vakzinzusammensetzung umfaßt:
(a) Ein im wesentlichen reines, immunogenes Polypeptid, das mindestens 900 Aminosäuren besitzt, umfassend ein Segment, das eine Aminosäuresequenz besitzt, die aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert; und
(b) einen pharmazeutisch geeigneten Träger.

36. Die Vakzinzusammensetzung gemäß Anspruch 35, wobei das Polypeptid in *N. meningitidis* vorhanden ist.

37. Die Vakzinzusammensetzung gemäß Anspruch 36, wobei das Polypeptid in den äußeren Membranen von *N. meningitidis* vorhanden ist.

38. Die Vakzinzusammensetzung gemäß Anspruch 35, wobei das Säugetier ein Mensch ist.

39. Monoklonale Antikörper erzeugt gegen ein im wesentlichen reines Polypeptid, das mindestens 900 Aminosäuren besitzt, umfassend ein Segment, das eine Aminosäuresequenz besitzt, die in *N. meningitidis* vorhanden ist, wobei die Aminosäuresequenz aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, oder antigene Fragmente solcher Polypeptide, wobei die antigenen Fragmente mindestens drei Wiederholungen der Hämolysin-Konsensussequenz LXGGXGNDX umfassen,
wobei X unabhängig voneinander jeglichen einzelnen Aminosäurerest repräsentiert.

40. Ein isoliertes Nukleinsäuremolekül, das ein Polypeptid kodiert, das mindestens 900 Aminosäuren besitzt, umfassend ein Segment, das eine Aminosäuresequenz besitzt, die in *N. meningitidis* vorhanden ist, wobei die Aminosäuresequenz aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, oder antigene Fragmente solcher Polypeptide, wobei die antigenen Fragmente mindestens drei Wiederholungen der Hämolysin-Konsensussequenz LXGGXGNDX umfassen,
wobei X unabhängig voneinander jeglichen einzelnen Aminosäurerest repräsentiert.

41. Ein Verfahren zum Detektieren der Anwesenheit von *N. meningitidis* in einer Probe, wobei das Verfahren die Schritte umfaßt:
(a) Herstellen einer Sonde, die ein Polypeptid erkennt, das ein Mitglied der Hämolysin-Familie der Toxine ist, oder ein Fragment davon, oder ein Nukleinsäuremolekül, das das Polypeptid oder ein Fragment kodiert, wobei das Polypeptid, das ein Mitglied der Hämolysin-Familie der Toxine ist, oder das Nukleinsäuremolekül, das das Polypeptid kodiert, in *N. meningitidis* vorhanden ist; und
(b) Bestimmen, ob die Sonde *N. meningitidis* in der Probe erkennt.

42. Das Verfahren gemäß Anspruch 41, wobei die Sonde ein Antikörper ist.

43. Das Verfahren gemäß Anspruch 42, wobei der Antikörper monoklonal ist.

44. Das Verfahren gemäß Anspruch 41, wobei die Sonde ein Nukleinsäuremolekül ist.

45. Ein Verfahren zum Detektieren der Anwesenheit von Antikörpern, die für ein Mitglied der Hämolysin-Familie der Toxine spezifisch sind, in einer Probe umfassend die Schritte:
(a) Herstellen eines antigenen Polypeptids, das mindestens 900 Aminosäuren besitzt, wobei das Polypeptid eine Aminosäuresequenz umfaßt, die in *N. miningitidis* vorhanden ist, umfassend ein Segment, das eine Aminosäuresequenz besitzt, die aus mindestens drei Wiederholungen der neun Aminosäuren langen Hämolysin-Konsensussequenz besteht, wobei die Hämolysin-Konsensussequenz aus mindestens vier der folgenden besteht:
L an Position 1;
G an Position 3;
G an Position 4;
G an Position 6;
N an Position 7;
D an Position 8; und
X an den Positionen 2, 5 und 9;
wobei X jeglichen einzelnen Aminosäurerest repräsentiert, und
(b) Bestimmen, ob das Polypeptid einen Antikörper in der Probe erkennt, oder antigene Fragmente solcher Polypeptide, wobei die antigenen Fragmente mindestens drei Wiederholungen der Hämolysin-Konsensussequenz LXGGXGNDX umfassen, wobei X unabhängig voneinander jeglichen einzelnen Aminosäurerest repräsentiert.

## Revendications

1. Polypeptide antigénique essentiellement pur, ayant au moins 900 acides aminés, comprenant un segment d'au moins cinquante résidus d'acides aminés et ayant une séquence d'acides aminés présente dans *N.meningitidis,* dans lequel la séquence d'acides aminés du segment est différente mais substantiellement homologue de la séquence d'acides aminés d'un segment d'un membre de la famille des hémolysines des toxines, et des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

2. Polypeptide selon la revendication 1, dans lequel le segment a au moins 100 résidus d'acides aminés.

3. Polypeptide selon la revendication 1, dans lequel le segment a au moins 200 résidus d'acides aminés.

4. Polypeptide selon la revendication 1, dans lequel la séquence d'acides aminés antigénique est immunogène.

5. Polypeptide selon la revendication 1, dans lequel la séquence d'acides aminés du polypeptide comprend la séquence représentée sur la figure 2.

6. Polypeptide selon la revendication 1, dans lequel des anticorps contre le polypeptide réagissent avec au moins un autre membre de la famille des hémolysines des toxines provenant d'autre genres de bactéries.

7. Polypeptide selon la revendication 6, dans lequel les autres genres de bactéries comprennent *Escherichia, Serratia, Pasteurella, Proteus*, *Actinobacillus, et Bordetella*.

8. Polypeptide selon la revendication 6, dans lequel les autres membres de la famille des hémolysines des toxines comprennent l'alpha-hémolysine provenant *d'Escherichia coli* ; la leucotoxine provenant *d'Actinobacillus actinomycetemcomitans*, la leucotoxine provenant de *Pasteurella haemolytica* ; l'adénylatecyclase provenant de *Bordetella pertussis* et l'adénylate-cyclase provenant de *Bacillus anthracis*.

9. Polypeptide selon la revendication 6, dans lequel l'autre membre de la famille des hémolysines des toxines est l'alpha-hémolysine provenant *d'E.coli.*

10. Procédé de production d'un antigène utile dans la protection d'un mammifère contre l'infection par *N. meningitidis*, comprenant les étapes consistant à :
(a) préparer un polypeptide antigénique essentiellement pur, ayant au moins 900 acides aminés et comprenant un segment ayant au moins cinquante résidus d'acides aminés, dans lequel la séquence d'acides aminés est différente, mais substantiellement homologue de la séquence d'acides aminés d'un segment d'un membre de la famille des hémolysines des toxines, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ;
(b) rendre le polypeptide non toxique pour les mammifères.

11. Procédé selon la revendication 10, dans lequel la séquence d'acides aminés est présente dans un polypeptide trouvé dans *N. meningitidis*.

12. Procédé selon la revendication 10, dans lequel le mammifère est un être humain.

13. Procédé de production d'une composition de vaccin utile pour protéger un mammifère de l'infection par *N. meningitidis*, comprenant les étapes consistant à :
(a) préparer un polypeptide antigénique essentiellement pur, ayant au moins 900 acides aminés, comprenant un segment ayant au moins cinquante résidus d'acides aminés, dans lequel la séquence d'acides aminés est différente, mais substantiellement homologue de la séquence d'acides aminés d'un segment d'un membre de la famille des hémolysines des toxines, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ;
(b) rendre le polypeptide non toxique pour les mammifères , et
(c) combiner le polypeptide non toxique à un support pharmaceutiquement acceptable.

14. Procédé selon la revendication 13, dans lequel le polypeptide est isolé de *N.meningitidis.*

15. Procédé selon la revendication 13, dans lequel le mammifère est un être humain.

16. Composition de vaccin capable d'immuniser les mammifères contre les infections par *N.meningitidis*, la composition de vaccin comprenant :
(a) un polypeptide immunogène ayant au moins 900 acides aminés, qui n'est pas toxique pour les mammifères et comprend un segment ayant au moins cinquante résidus d'acides aminés, dans lequel la séquence d'acides aminés est différente mais substantiellement homologue de' la famille des hémolysines des toxines, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ; et
(b) un support pharmaceutiquement acceptable.

17. Composition de vaccin selon la revendication 16, dans laquelle le polypeptide est présent dans *N. meningitidis*.

18. Composition de vaccin selon la revendication 17, dans laquelle le polypeptide est présent dans les membranes extérieures de *N.meningitidis*.

19. Composition de vaccin selon la revendication 16, dans laquelle le mammifère est un être humain.

20. Anticorps monoclonaux élevés contre un polypeptide ayant au moins 900 acides aminés, comprenant un segment d'au moins cinquante résidus d'acides aminés et ayant une séquence d'acides aminés présente dans *N.meningitidis*, dans lequel la séquence d'acides aminés du segment est différente, mais substantiellement homologue de la séquence d'acides aminés d'un segment d'un membre de la famille des hémolysines des toxines, et des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

21. Molécule d'acide nucléique isolée qui code un polypeptide ayant au moins 900 acides aminés, comprenant un segment d'au moins cinquante résidus d'acides aminés et ayant une séquence d'acides aminés présente dans *N. meningitidis*, dans lequel la séquence d'acides aminés du segment est différente, mais substantiellement homologue de la séquence d'acide aminé d'un segment d'un membre de la famille des hémolysines des toxines, et des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

22. Procédé de détection de la présence d'anticorps spécifiques pour un membre de la famille des hémolysines des toxines dans un échantillon , comprenant les étapes consistant à :
(a) préparer un polypeptide antigénique, essentiellement pur, ayant au moins 900 acides aminés comprenant un segment ayant au moins cinquante résidus d'acides aminés et dans lequel le polypeptide comprend une séquence d'acides aminés présente dans *N.meningitidis,* dans lequel la séquence d'acides aminés du segment est différente mais substantiellement homologue de la séquence d'acides aminés d'un segment d'un membre de la famille des hémolysines des toxines, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ; et
(b) déterminer si le polypeptide reconnaît un anticorps dans l'échantillon.

23. Polypeptide essentiellement pur ayant au moins 900 acides aminés, comprenant un segment ayant une séquence d'acides aminés présente dans *N.meningitidis,* dans lequel la séquence d'acides aminés consiste en au moins trois répétitions de la séquence consensus d'hémolysine à neuf acides aminés, la' séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans lequel x représente n'importe quel résidu d'acide aminé simple, et des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

24. Polypeptide selon la revendication 23, dans lequel le segment a au moins 100 résidus d'acides aminés.

25. Polypeptide selon la revendication 23, dans lequel le segment a au moins 200 résidus d'acides aminés.

26. Polypeptide selon la revendication 23, dans lequel la séquence d'acides aminés antigénique est immunogène.

27. Polypeptide selon la revendication 23, dans lequel les anticorps contre le polypeptide réagissent avec au moins un autre membre de la famille des hémolysines des toxines provenant d'autres genres de bactéries.

28. Polypeptide selon la revendication 23, dans lequel les autres genres de bactéries comprennent *Escherichia, Serratia, Pasteurella, Proteus, Actinobacillus et Bordetella.*

29. Polypeptide selon la revendication 27, dans lequel les autres membres de la famille des hémolysines des toxines comprennent l'alpha-hémolysine provenant de *Escherichia coli,* la leucotoxine de *Actinobacillus actinomycetemcomitans,* la leucotoxine provenant de *Pasteurella haemolytica,* l'adénylate-cyclase provenant de *Bordetella* pertussis, et l'adénylate-cyclase provenant de *Bacillus anthracis.*

30. Polypeptide selon la revendication 27, dans lequel l'autre membre de la famille des hémolysines des toxines est l'alpha-hémolysine provenant de *E.coli.*

31. Procédé de production d'un antigène utile dans la protection d'un mammifère contre l'infection par *N.meningitidis,* comprenant les étapes consistant à :
(a) préparer un polypeptide essentiellement pur ayant au moins 900 acides aminés comprenant un segment ayant une séquence d'acides aminés qui consiste en au moins trois répétitions de la séquence consensus d'hémolysine à neuf acides aminés, la séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans lequel X représente n'importe quel résidu d'acide aminé simple, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ; et
(b) rendre le polypeptide ou fragment non toxique pour les mammifères.

32. Procédé selon la revendication 31, dans lequel la séquence d'acides aminés est présente dans un polypeptide trouvé dans *N.meningitidis.*

33. Procédé de production d'une composition de vaccin utile pour protéger un mammifère de l'infection par *N.meningitidis*, comprenant les étapes consistant à :
(a) préparer un polypeptide antigénique, essentiellement pur, ayant au moins 900 acides aminés, comprenant un segment ayant une séquence d'acides aminés qui consiste en au moins trois répétitions de la séquence consensus d'hémolysine à neuf acides aminés, la séquence consensus d'hémolysine consistant en au moins quatre des
L en position 1 ;
G en position 3 ;
G en position 4 ;
G en position 6 ;
N en position 7 ;
D en position 8 ; et
X en positions 2, 5 et 9 ;
dans laquelle X représente n'importe quel résidu d'acide aminé simple, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ;
(b) rendre le polypeptide non toxique pour les mammifères ; et
(c) combiner le polypeptide non toxique avec un support pharmaceutiquement acceptable.

34. Procédé selon la revendication 33, dans lequel le polypeptide est isolé *N. meningitidis.*

35. Composition de vaccin capable d'immuniser les mammifères contre les infections par *N.meningitidis,* la composition de vaccin comprenant :
(a) un polypeptide immunogène essentiellement pur, ayant au moins 900 acides aminés, comprenant un segment ayant une séquence d'acides aminés qui consiste en au moins trois répétitions de la séquence consensus d'hémolysine à neuf acides aminés, la séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans laquelle X représente n'importe quel résidu d'acide aminé simple, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple ; et
(b) un support pharmaceutiquement acceptable.

36. Composition de vaccin selon la revendication 35, dans laquelle le polypeptide est présent dans *N.meningitidis.*

37. Composition de vaccin selon la revendication 36, dans laquelle le polypeptide est présent dans les membranes extérieures de *N.meningitidis.*

38. Composition de vaccin selon la revendication 35, dans laquelle le mammifère est un être humain.

39. Anticorps monoclonaux élevés contre un polypeptide essentiellement pur, ayant au moins 900 acides aminés, comprenant un segment ayant une séquence d'acides aminés présente dans *N.meningitidis,* dans lequel la séquence d'acides aminés consiste en au moins trois répétitions de la séquence consensus d'hémolysine à neuf acides aminés, la séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans laquelle X représente n'importe quel résidu d'acide aminé simple, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

40. Molécule d'acide nucléique isolée qui code un polypeptide ayant au moins 900 acides aminés, comprenant un segment ayant une séquence d'acides aminés présente dans *N.meningitidis,* dans lequel la séquence d'acides aminés consiste en au moins trois répétitions de la séquence consensus d'hémolysine à neuf acides aminés, la séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans laquelle X représente n'importe quel résidu d'acide aminé simple, ou des fragments antigéniques de ces polypeptides, dans lesquels les fragments antigéniques comprennent au moins trois répétitions de la séquence consensus d'hémolysine LXGGXGNDX, dans laquelle X, indépendamment, représente n'importe quel résidu d'acide aminé simple.

41. Procédé de détection de .la présence de *N.meningitidis* dans un échantillon, comprenant les étapes consistant à :
(a) préparer une sonde qui reconnaît un polypeptide qui est un membre de la famille des hémolysines des toxines ou un fragment de celui-ci, ou une molécule d'acide nucléique codant le polypeptide ou un fragment, dans lequel le polypeptide qui est membre de la famille des hémolysines des toxines ou une molécule d'acide nucléique codant le polypeptide est présent dans *N.meningitidis;* et
(b) déterminer si la sonde reconnaît *N.meningitidis* dans l'échantillon.

42. Procédé selon la revendication 41, dans lequel la sonde est un anticorps.

43. Procédé selon la revendication 42, dans lequel l'anticorps est monoclonal.

44. Procédé selon la revendication 41, dans lequel la sonde est une molécule d'acide nucléique.

45. Procédé de détection de la présence d'anticorps spécifiques pour un membre de la famille des hémolysines des toxines dans un échantillon, comprenant les phases étapes consistant à :
(a) préparer un polypeptide antigénique ayant au moins 900 acides aminés et dans lequel le polypeptide comprend une séquence d'acides aminés présente dans *N.meningitidis,* comprenant un segment ayant une séquence d'acides aminés qui consiste en au moins trois répétitions de la séquence consensus d'hémolysine à neuf acides aminés, la séquence consensus d'hémolysine consistant en au moins quatre des :
L en position 1 ;
G en position 3 ;
G en position 4 ;
G en position 6 ;
N en position 7 ;
D en position 8 ; et
X en positions 2, 5 et 9 ;
dans laquelle X représente n'importe quel résidu d'acide aminé simple ; et
(b) déterminer si le polypeptide reconnaît un anticorps dans l'échantillon, ou des fragments antigéniques de ces polypeptides, dans lesquels les fragments antigéniques comprennent au moins trois répétitions de la séquence consensus d'hémolysine LXGGXGNDX dans laquelle X, indépendamment, représente n'importe quel résidu d'acide aminé simple.
